(19) Europäisches Patentamt
European Patent Office
Office européen des brevets

(11) Publication number: **0 020 052**
**B1**

(12) **EUROPEAN PATENT SPECIFICATION**

(45) Date of publication of patent specification: **20.07.83**

(21) Application number: **80301572.6**

(22) Date of filing: **14.05.80**

(51) Int. Cl.³: **C 07 C 79/46,**
C 07 C 103/82,
C 07 C 103/84,
C 07 C 121/75,
C 07 C 153/09,
A 01 N 37/48,
C 07 C 93/20,
C 07 C 103/16,
C 07 C 103/34

(54) **Novel substituted nitrodiphenyl ethers, herbicidal compositions containing them, processes for the preparation thereof and the use thereof for combating weeds.**

(30) Priority: **16.05.79 US 39471**
**17.12.79 US 104598**

(43) Date of publication of application:
**10.12.80 Bulletin 80/25**

(45) Publication of the grant of the patent:
**20.07.83 Bulletin 83/29**

(84) Designated Contracting States:
**AT BE CH DE FR GB IT LI LU NL SE**

(56) References cited:
**EP - A - 0 033 629**
**EP - A - 0 040 898**
**DE - A - 2 501 797**
**DE - A - 2 753 900**
**DE - A - 3 029 728**
**US - A - 3 798 276**
**US - A - 3 928 416**
**US - A - 4 070 178**

(73) Proprietor: **Rohm and Haas Company**
**Independence Mall West**
**Philadelphia, Pennsylvania 19105 (US)**

(72) Inventor: **Johnson, Wayne Orrin**
**346 Centennial Road**
**Warminster, Pennsylvania, 18974 (US)**

(74) Representative: **Wood, Peter Worsley Spencer et al,**
**Rohm and Haas Company Patent Department**
**Chesterfield House Bloomsbury Way**
**London WC1A 2TP (GB)**

**EP 0 020 052 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Novel substituted nitrodiphenyl ethers, herbicidal compositions containing them, processes for the preparation thereof and the use thereof for combating weeds.

This invention concerns the provision of new nitrodiphenyl ethers, the preparation thereof, herbicidal compositions containing them and methods of combating weeds.

Diphenyl ethers are known to be effective weed control agents. See for example U.S. Patent Nos. 3,928,416; 3,454,392; 3,798,276; 3,873,303; 4,001,005, 4,029,493, OLS 273 53 900 and Japanese Patent Publication No. 49—66828 among the many patent specifications published. However, herbicidal effectiveness of a diphenyl ether cannot be predicted from its structure only and often quite closely related compounds will have quite different weed control abilities. See, *Advances in Agronomy,* Vol. 24, pages 331, 332, 355, 356, 357 and 358, *Herbicides, Chemical Degradation and Mode of Action*, Kearney and Kaufman, Vol. 2, Dekker, Inc. pages 552—563 and 728—737 and *Mode of Action of Herbicides,* Ashton and Crafts and also U.S. Patent Nos. 3,454,392 and 3,776,961.

Of the above cited Patent Specifications, the OLS discloses herbicidal substituted 4-trifluoromethyl-4-nitrodiphenyl ethers in which the 2 position is occupied by hydrogen, halogen, tri-halomethyl, alkyl or cyano, the 6 position is occupied by hydrogen, halogen or trihalomethyl and the 3' position is occupied by a group of the formula $—CO_2Z'$ or CONRZ' where Z' is alkenyl, haloalkenyl, alkynyl, haloalkynyl, optionally substituted aralkenyl, cycloalkenyl or epoxyalkyl. Also the Japanese patent specification discloses herbicidal substituted nitrodiphenyl ethers of the formula:

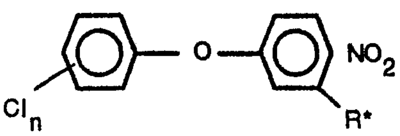

where n is 1, 2 or 3 and R* can be any one of a large number of groups including alkoxycarbonyl-alkoxycarbonyl such as, in the case where $Cl_n$ is 4-chloro, $—CO_2CH_2CO_2C_2H_5$.

An ideal herbicide should give selected weed control, over the full growing season, with a single administration at low rates of application. It should be able to control all common weeds by killing them as the seed, the germinating seed, the seedling, and the growing plant. At the same time, the herbicide should be substantially non-phytotoxic to the crops to which it is applied and should decompose or otherwise be dissipated so as not to poison the soil permanently. The known diphenyl ether herbicides fall short of these ideals and thus the search has continued to discover new herbicides which are more selective or which complement the activities of known diphenyl ethers.

The new nitrodiphenyl ethers concerned in the invention are, in comparison with known herbicides, of utility in one or more of the following respects, viz. acceptable lack of phytotoxicity towards plants yielding an agronomic crop, broad spectrum weed control and acceptable toxicological properties.

In accordance with the present invention, there is provided a new class of diphenyl ether herbicides having the following structural formula:

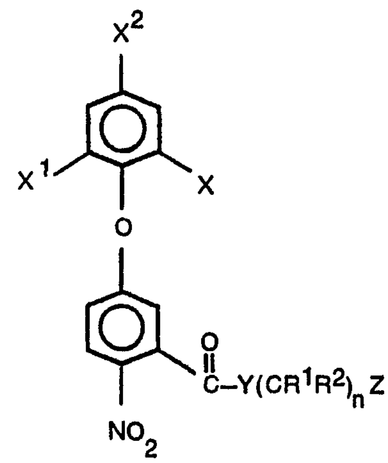

(I)

wherein X is hydrogen, halo, trihalomethyl (preferably trifluoromethyl), alkyl (e.g. $(C_1—C_{10})—$, $(C_1—C_6)—$ and, preferably, $(C_1—C_4)$alkyl), cyano or nitro;

$X^1$ is hydrogen, halo or trihalomethyl (preferably trifluoromethyl).

$X^2$ is trihalomethyl (preferably trifluoromethyl)

Y is O, NH, $NR^1$ or S;

$R^1$ and $R^2$ are the same or different and are selected from hydrogen, $(C_1—C_4)$alkyl and phenyl-$(C_1—C_4)$ alkyl (preferably benzyl or phenethyl);

n is an integer of from 1 to 5 (the groups $-CR^1R^2$ being the same or different when n=>1); and Z is carboxy, amino, mono- or di- $(C_1-C_4)$ alkylamino and the agronomically acceptable salts, esters and amides of compounds wherein Z is carboxy.

The compounds of the invention are distinguished from those of the above cited Japanese Patent Specification in that they contain a 4-trihalomethyl substituent in place of chlorine, there being nothing in the prior art to motivate the replacement of a 4-chloro substituent with a trihalomethyl substituent. In the case of the above cited OLS, there was again nothing in the prior art to motivate the replacement of the 3-3-substituent, $CO_2Z'$ or CONRN', with applicant's

$$-\overset{\overset{\displaystyle O}{\|}}{C}Y(CR^1R^2)_nZ \text{ 3'-substituent.}$$

Examples of the salts when Z is carboxy include the alkali metal and alkaline earth metal salts, such as lithium, sodium, potassium, calcium, barium, strontium and magnesium. Examples of such esters include alkyl (preferably $C_1-C_5$, $C_6$ or $C_7$) optionally substituted with halo, particularly fluoro to form, for example, a $CF_3$ group or moiety; alkoxyalkyl (preferably $(C_1-C_4)$ alkoxy $(C_1-C_6)$ alkyl optionally substituted with halo in the alkoxy moiety particularly fluoro to form, for example, a $CF_3$ group or moiety; alkenyl (preferably $C_3-C_5$) such as allyl, $\alpha$-methylallyl or $\alpha$-ethylallyl); and alkynyl (preferably $C_3-C_5$ such as propynyl. Examples of amides are those in which the amido nitrogen atom contains one or two alkyl substituents, preferably $(C_1-C_4)$ alkyl such as methyl.

One sub-class of compounds of Formula I are those in which X is hydrogen, halo, trifluoromethyl, $(C_1-C_4)$ alkyl, cyano or nitro, X preferably being chloro; $X^1$ is hydrogen, halo or trifluoromethyl, $X^1$ preferably being hydrogen; $X^2$ is trifluoromethyl; Y is O, NH or S (preferably O); $R^1$ and $R^2$ are the same or different and are selected from hydrogen, $(C_1-C_4)$ alkyl and phenyl$(C_1-C_4)$alkyl; n is 1 to 5 (preferably 1 to 3 and more preferably 1 or 2); Z is as defined for Formula I (but is preferably $-CO_2(C_1-C_4)$ alkyl); and (when Z is carboxy) the agronomically acceptable salts, esters and amides.

The term "halo" used in this specification means, unless otherwise defined, bromo, chloro, fluoro or iodo. The terms alkyl, alkoxy, alkenyl and alkynyl include all straight and branched chain groups (i.e. all isomeric forms) so that, for example, $(C_1-C_4)$ alkyl means $CH_3$, $C_2H_5$, $C_3H_7$, $C_4H_9$ and all isomers of the last two groups.

Another sub-class of compounds provided by this invention is represented by the formula:

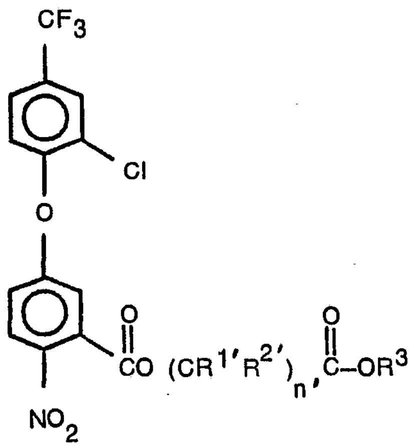

(IA)

wherein $R^{1\prime}$ and $R^{2\prime}$ are the same or different and are selected from hydrogen and $(C_1-C_4)$ alkyl, n' is 1 or 2 and $R^3$ is $(C_1-C_4)$ alkyl.

Individual compounds of Formula I are those wherein (1), X is chlorine, $X^1$ is hydrogen, $X^2$ is trifluoromethyl, Y is oxygen, $R^1$ and $R^2$ are hydrogen, n is 1 and Z is $CO_2CH_3$, $CO_2$-t-$C_4H_9$, $CO_2H$, $CO_2CH_2CH_2OCH_3$, $CO_2CH_2CF_3$, $CO_2CH_2CH{=}CH_2$ or $CON(CH_3)_2$, (2) X is chlorine, $X^1$ is hydrogen, $X^2$ is trifluoromethyl, Y is oxygen, $R^1$ is methyl, $R^2$ is hydrogen, n is 1 and Z is $CO_2CH_3$ or $CO_2C_2H_5$ (3) X is chlorine, $X^1$ is hydrogen, $X^2$ is trifluoromethyl, Y is oxygen, $R^1$ and $R^2$ are hydrogen, n is 3 and Z is $CO_2C_2H_5$ or (4) X is chlorine, $X^1$ is hydrogen, $X^2$ is trifluoromethyl, Y is oxygen, $R^1$ and $R^2$ are methyl, n is 1 and Z is $CO_2C_2H_5$) (5) X is chlorine, $X^1$ is hydrogen, $X^2$ is trifluoromethyl, Y is oxygen, $R^1$ is $CH_3$, $R^2$ is H, n is 1 and Z is $CO_2CH_3$, (6) X is chlorine, $X^1$ is hydrogen, $X^2$ is trifluoromethyl, Y is oxygen, $R^1$ and $R^2$ are hydrogen, n is 1 and Z is $CO_2Na$, $CO_2K$ or $CO_2Li$, (7) X is chlorine, $X^1$ is hydrogen, $X^2$ is trifluoromethyl, Y is oxygen, $R^1$ is benzyl, $R^2$ is hydrogen, n is 1 and Z is $CO_2CH_3$, (8) X is chlorine, $X^1$ is hydrogen, $X^2$ is trifluoromethyl, Y is O, S or NH, $R^1$ is hydrogen (when Y is S), $C_2H_5$ (when Y is O) or $CH_3$ or $i$-$C_3H_7$ (when Y is NH), $R^2$ is hydrogen, n is 1 and Z is $CO_2C_2H_5$, (9) X is chlorine, $X^1$ is hydrogen, $X^2$ is trifluoromethyl, Y is oxygen, $R^1$ is methyl, $R^2$ is hydrogen, n is 1 and Z is $CONH_2$, $CO_2H$, $CO_2Na$ or $CO_2K$, or (10) X is

chlorine, $X^1$ is hydrogen, $X^2$ is trifluoromethyl, Y is oxygen, $—(CR^1R^2)_n—$ is $—(CH_2)_4—$ and Z is $CO_2C_2H_5$.

Further individual compounds of Formula I are those wherein X is chlorine, $X^1$ is hydrogen, $X^2$ is trifluoromethyl, Y is oxygen and, Z is $CO_2CH_3$ or $CO_2C_2H_5$, when Z is $CO_2CH_3$, $—(CR^1R^2)_n—$ being $—(CH_2)_2—$ or $—CH(CH_3)CH_2—$ and, when Z is $—CO_2C_2H_5$, $—(CR^1R^2)_n—$ being $—C(CH_3)_2CH_2—$, $—CH(CH_3)CH(CH_3)—$, $—(CH_2)_2—$ or $—(CH_2)_3—$ in which one of the hydrogen atoms is replaced by $CH_3$.

Individual compounds of Formula IA are those wherein $—(CR^{1'}R^{2'})_{n'}—$ is $—CH_2—$ or $—CH(CH_3)CH_2—$ and, $R^3$ is $C_2H_5$.

Examples of the compounds of the invention embraced by Formula I include:

EXEMPLARY LIST

2-chloro-4-trifluoromethyl-3′-(methoxycarbonylmethoxycarbonyl)-4′-nitrodiphenyl ether,
2-chloro-4-trifluoromethyl-3′-(sodiocarboxymethoxycarbonyl)-4′-nitrodiphenyl ether,
2-fluoro-4-trifluoromethyl-3′-{(1-methoxycarbonyl)-n-propylthiocarbonyl}-4′-nitrodiphenyl ether,
2-fluoro-4-trifluoromethyl-6-chloro-3′-{(1-allyloxycarbonyl)-n-butoxycarbonyl}-4′-nitrodiphenyl ether,
2-bromo-4-trifluoromethyl-3′-{(2-ethoxycarbonyl)ethoxycarbonyl}-4′-nitrodiphenyl ether,
2-methyl-4-trifluoromethyl-3′-{(1-methoxycarbonyl)phenethoxycarbonyl}-4′-nitrodiphenyl ether, and
2-cyano-4-trifluoromethyl-3′-{ω-(methoxycarbonyl)-n-pentyloxycarbonyl}-4′-nitrodiphenyl ether.

The compounds of Formula I may be prepared by methods described in the literature for compounds of analogous structure or by methods specially devised for the purpose. It is, however, preferred to (1) react an acid halide with an alkoxy-substituted compound, (2) react an alkali metal or alkaline earth metal carboxylate with a halo substituted compound, (3), where necessary, react an appropriate intermediate with a nitrating agent or (4) react a metal phenate with an appropriately substituted 5-halo-2-nitrobenzene.

A first process embodiment comprises reacting a 4-substituted phenyl-3′-halocarbonyl-4′-nitrophenyl ether (II *infra*) with an appropriate compound in the presence of an inert solvent (e.g. toluene) and optionally in the presence of an acid acceptor. The reaction may be conducted at a temperature in the range of from about 20° to about 150°C and the reaction period may vary from about 15 minutes to about 24 hours; however, the reaction is generally conducted at 30° to 40°C for a period of time of about 2 hours. The following reaction scheme illustrates this process:

(II)

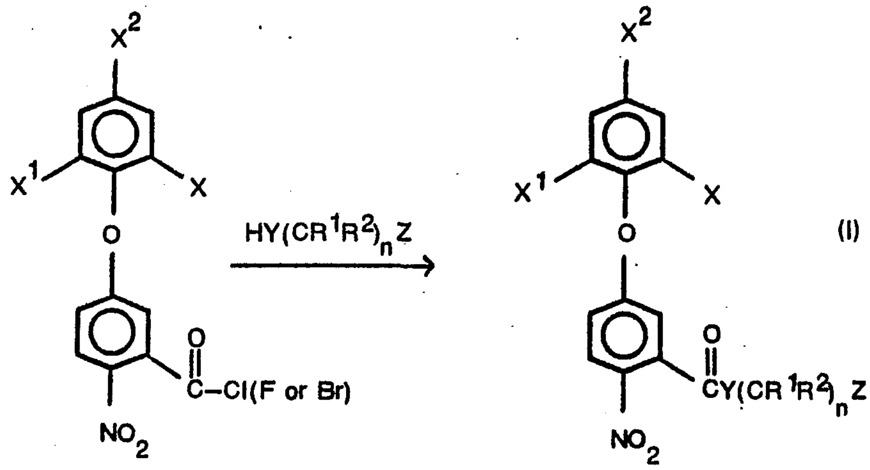


(I)

wherein X, $X^1$, $X^2$, Y, $R^1$, $R^2$, n and z are as defined in connection with Formula I.

A second process embodiment comprises reacting a 4-substituted phenyl-3′-alkali metal (or alkaline earth metal) oxycarbonyl-4′-nitrophenyl ether with a halo-substituted compound in the presence of an inert solvent such as dimethyl formamide, dimethyl sulfoxide, sulfolane or methylethyl ketone. This reaction can, for example, be carried out at a temperature in the range of from about 20°

to about 150°C and the reaction period may vary from about 1/4 to about 6 hours. The following reaction scheme illustrates this process:

(III)

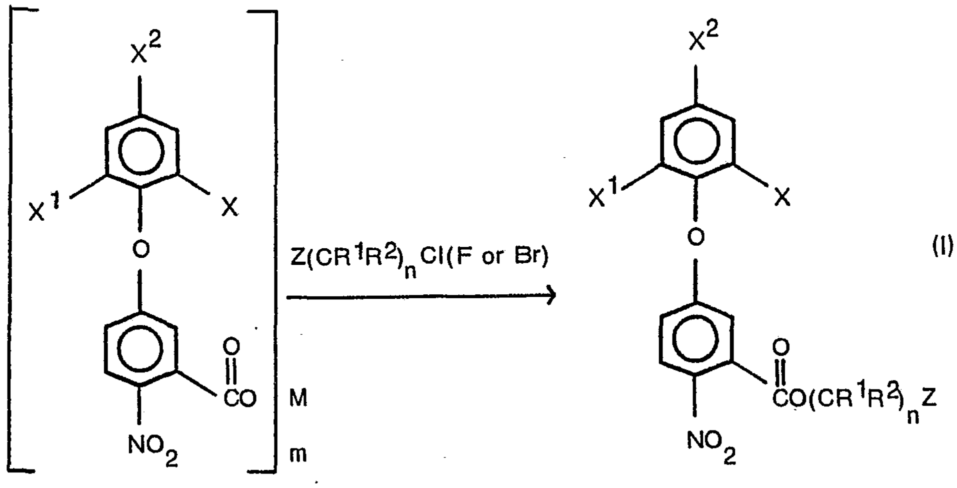


(I)

wherein X, $X^1$, $X^2$, $R^1$, $R^2$, n and Z are as defined in connection with Formula I, M is a cation derived from an alkali or alkaline earth metal, and m is 1 or 2.

An alternative process comprises reacting an appropriately substituted phenate with an appropriately substituted 5-halo-2-nitrobenzene. The following reaction scheme illustrates this process:

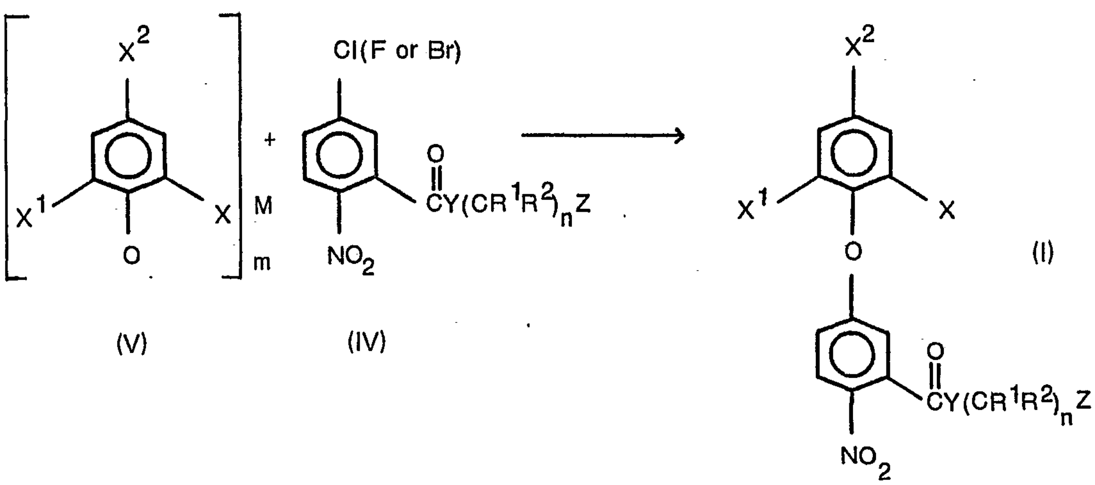

(V) (IV) (I)

wherein X, $X^1$, $X^2$, Y, $R^1$, $R^2$, n, Z and M are as defined for Formulae I and III and m is 1 or 2. The reaction is generally conducted at a temperature in the range of from about 50° to about 150°C and the reaction period may vary from about 1 to about 96 hours. Solvents which may be employed include dimethyl sulfoxide, dimethyl formamide, sulfolane, and methylethyl ketone.

A fourth process embodiment comprises reacting a 4-substituted phenyl-3'-alkali metal (or alkaline earth metal) oxycarbonyl phenyl ether with a halo substituted compound as described in the above second process embodiment, followed by nitration employing, for example, as the nitrating agent, nitric acid, nitric acid/sulfuric acid, nitroniumtetrafluoroborate, nitroniumhexafluorophosphate or acetyl nitrate. Solvents, if employed include sulfolane, acetic anhydride or acetyl nitrate. The temperature used may be in the range of from about 20° to about 200°C and is preferably in the range

of from about 50° to about 150°C. The reaction period may range from 1/2 to 6 hours. The following reaction scheme illustrates this process:

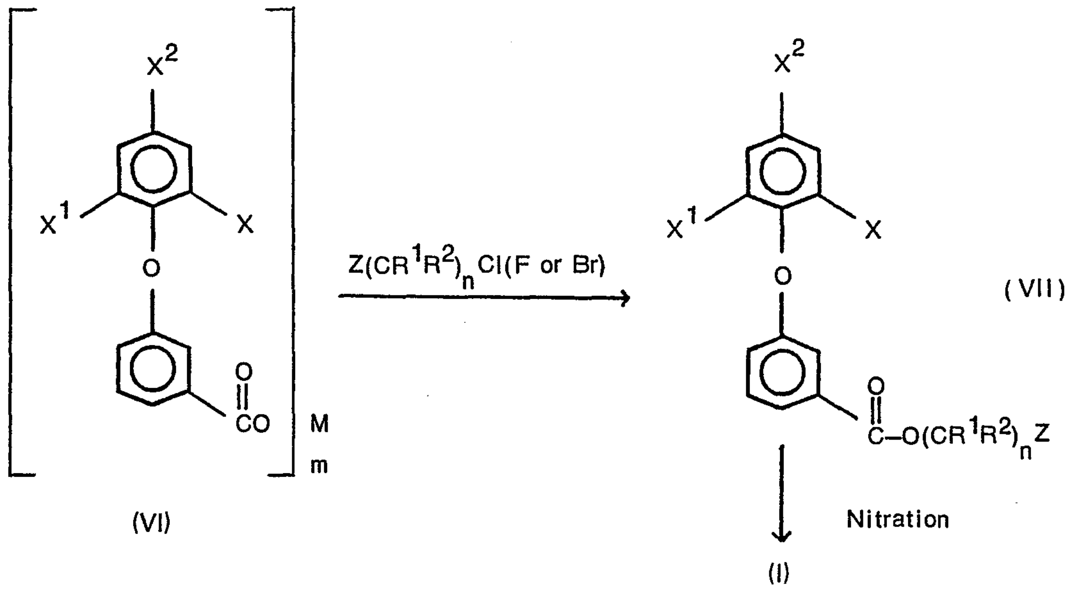


(VI) (VII) (I)

wherein X, $X^1$, $X^2$, $R^1$, $R^2$, n and z are as defined in connection with Formulae I and III.

The following examples are given by way of illustration. Percentages are on a weight basis unless otherwise noted. Analytical data for the most of the compounds prepared in accordance with the examples are given in Table II which follows the examples.

Example 1

2-Chloro-4-trifluoromethyl-3′-(*tert*/butoxycarbonylmethoxycarbonyl)-4-nitrodiphenyl ether

2-Chloro-4-trifluoromethyl-3′-carboxy-4-nitrodiphenyl ether (46.2 g; 0.128 mole) was dissolved in dimethyl sulfoxide (100 ml). Potassium carbonate (17.7 g; 0.128 mole) was added and the reaction mixture was heated with stirring to 60°C. The reaction mixture was then cooled to 15°C and allowed to warm to room temperature. *Tert*-butyl bromoacetate was then added and the reaction mixture stirred at room temperature for one hour. The above reaction procedure was repeated and the products of the two reactions were combined and diluted with ether, the ether extract being washed with water, then with aqueous 1% sodium and finally with aqueous 5% sulfuric acid. The ether solution was then dried over anhydrous magnesium sulfate. The dried ether solution was filtered through activated silica gel and the ether evaporated to afford 104.8 g of 2-chloro-4-trifluoromethyl-3′-(*tert*-butoxymethoxy-carbonyl)-4′-nitrodiphenyl ether which after recrystallization from hexane at 0°C had a m.p. of 25°C. Elemental analysis: Found, C, 50.13; H, 3.51; N, 2.87; Cl, 7.38; F, 12.30. $C_{20}H_{17}ClF_3NO_7$ requires C, 50.49; H, 3.60; N, 2.94; Cl, 7.45; F, 11.98.

Example 2

2-Chloro-4-trifluoromethyl-3′-(carboxymethoxycarbonyl)-4-nitrodiphenyl ether

2-Chloro-4-trifluoromethyl-3′-*tert*-butoxycarbonyl-methoxycarbonyl-4-nitrodiphenyl ether (12.0 g) was heated in an oil bath to 165°C until bubbling ceased. The product was dissolved in ether, extracted into 5% aqueous potassium carbonate, the extract acidified with 5% aqueous sulfuric acid and then extracted with ether. The ether extract was dried over anhydrous magnesium sulfate, filtered through activated silica gel and the ether removed to afford 9.9 g of 2-chloro-4-trifluoromethyl-3′-(carboxymethoxycarbonyl)-4′-nitrodiphenyl ether. The product was recrystallized from toluene/hexane, m.p. 103.5°—106°C. Found: C, 45.99; H, 2.59; N, 2.88; Cl, 8.00; F, 13.02; $C_{16}H_7ClF_3NO_7$ requires C, 45.79 H, 2.16; N, 3.34; Cl 8.45; F, 13.58.

Example 3

2-Chloro-4-trifluoromethyl-3′-{(1-methoxycarbonyl)ethoxycarbonyl}-4′-nitrodiphenyl ether

2-Chloro-4-trifluoromethyl-3′-carboxy-4′-nitrodiphenyl ether (10.0 g; 0.028 mole) was dissolved in methylethyl ketone (150 ml) and potassium carbonate (3.9 g; 0.028 mole) was added. The solution was heated with stirring to reflux and a white solid precipitated. The solution was heated with stirring to reflux and a white solid precipitated. The solution was cooled to room temperature and methyl $\alpha$-bromopropionate (5.7 g) was added. The reaction mixture was heated to 75°C for approximately 3 hours during which period additional methyl $\alpha$-bromopropionate (1.0 g) was added. The reaction mixture was diluted with ether and the aqueous layers separated and discarded. The ether extract was

washed with aqueous 5% sulfuric acid and then with aqueous 1% sodium hydroxide solution. The ether was dried over anhydrous magnesium sulfate, filtered through silica gel and the ether removed to afford 8.3 g of 2-chloro-4-trifluoromethyl-3-(1-carbomethoxyethoxycarbonyl)-4′-nitrodiphenyl ether as an oil.

## Example 4

2-Chloro-4-trifluoromethyl-3′-{(1-ethoxycarbonyl)ethoxycarbonyl}-4′-nitrodiphenyl ether

To a solution of 2-chloro-4-trifluoromethyl-3′-chlorocarbonyl-4′-nitrophenyl ether (9.0 g; 0.0236 mole) in 1,2-dimethoxyethane (25 ml) in a 50 ml round bottom flask was added at room temperature, ethylacetate (2.8 g; 0.0236 mole) and pyridine (1.58 g; 0.0236 mole). The reaction mixture was stirred over the week-end at room temperature, and then heated to 75°C for about 24 hours. The reaction mixture was filtered to remove solid and the filtrate concentrated to afford 12 g of a yellow oil. The yellow oil was chromatographed over 100 g of silica gel using toluene as the solvent to give 3.5 g of 2-chloro-4-trifluoromethyl-3′-(1-carboethoxy)ethoxycarbonyl-4′-nitrodiphenyl ether as a yellow oil of 74% purity contaminated with a second product.

## Example 5

2-Chloro-4-trifluoromethylphenyl-3′-2{(N,N-dimethylamino)ethoxycarbonyl}-4′-nitrophenyl ether

*Step A* — Potassium 2-chloro-4-trifluoromethylphenoxide

2.Chloro-4-trifluoromethylphenol (1.96 g; 0.010 mole) was dissolved in dimethyl sulfoxide (15 ml) and potassium carbonate (2.76 g; 0.020 mole) was added. The reaction mixture was stirred at room temperature overnight to afford potassium 2-chloro-4-trifluoromethylphenoxide.

*Step B* — 2-Chloro-4-trifluoromethylphenyl-3′-2{(N,N-dimethylamino)ethoxycarbonyl}-4′-nitrophenyl ether

To the product of Step A was added 5-fluoro-2-nitro(N,N-dimethylaminoethoxycarbonyl)benzene (2.93 g). The reaction mixture was stirred at room temperature for 3 hours and then warmed to 45°C for about 18 hours. The reaction mixture was poured into carbon tetrachloride (100 ml) and extracted with two portions of water (2 x 50 ml). The carbon tetrachloride layer was dried over anhydrous sodium sulfate, filtered and the solvent removed under vacuum to afford 2.8 g of 2-chloro-4-trifluoromethylphenyl-3′-2-{(N,N-dimethylamino)ethoxycarbonyl}-4′-nitrophenyl ether. Recrystallization from benzene/hexane afforded substantially pure product having a melting point of 70°—71.5°C. Elemental analysis: Found C 48.94; H, 3.74; N, 6.18; Cl, 8.14; F, 13.58; $C_{18}H_{16}ClF_3N_2O_5$ requires C, 49.95; H, 3.73; N, 6.47; Cl, 8.19; F, 13.14.

The diphenyl ethers identified in Table I below are, following substantially the procedures of Examples 1 to 3 also prepared in accordance with the reaction scheme preceding the table.

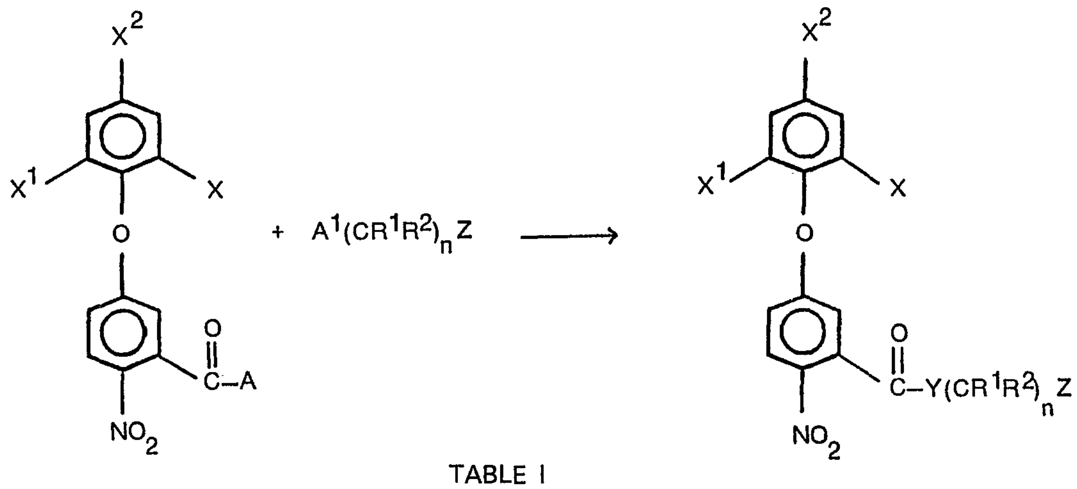

TABLE I

| Ex. No. | X | $X^1$ | $X^2$ | Y | A | $A^1$ | $R^1$ | $R^2$ | n | Z |
|---|---|---|---|---|---|---|---|---|---|---|
| 6 | Cl | H | $CF_3$ | S | Cl | SH | H | H | 1 | $CO_2CH_3$ |
| 7 | Cl | H | $CF_3$ | NH | Cl | $NH_2$ | H | H | 1 | $CO_2CH_3$ |
| 8 | Cl | H | $CF_3$ | O | OK | Cl | H | H | 2 | $CO_2H$ |
| 9 | Cl | H | $CF_3$ | O | OK | Br | H | H | 3 | $CO_2C_2H_5$ |
| 10 | Cl | H | $CF_3$ | O | OK | Br | $—CH_2C_6H_5$ | H | 1 | $CO_2CH_3$ |

Example 11

2-Chloro-4-trifluoromethyl-3'-{(1-ethoxycarbonyl)isopropoxycarbonyl}-4'-nitrodiphenyl ether

2-Chloro-4-trifluoromethyl-3'-carboxy-4'-nitrodiphenylether (9.04 g) and anhydrous potassium carbonate (6.91 g) in dimethylsulfoxide (30 ml) were heated to 75°C and ethyl $\alpha$-bromoisobutyrate (4.9 g) in dimethyl sulfoxide (10 ml) was added and heating continued for 2 hours at 80—82°C, at which point additional ethyl $\alpha$-bromoisobutyrate (0.5 g) was added. After two additional hours of heating, the mixture was cooled and diluted with ether. The ether solution was washed with aqueous 5% $H_2SO_4$, then with aqueous 1% NaOH, dried over anhydrous magnesium sulfate, filtered through activated silica gel and finally thoroughly stripped at 0.5 mm/60—65°C, of unreacted bromoester. The residue solidified to a yellow powder which was recrystallized from hexane to afford the desired ester, yield 1.8 g, m.p. 92°—94.5°C. Found: C, 50.15; H, 3.54; N, 2.89; Cl, 7.54; F, 11.85. $C_{20}H_{17}ClF_3NO_7$ requires C, 50.45; H, 3.60; N, 2.94; Cl, 7.45; F, 11.98.

Example 12

2-Chloro-4-trifluoromethylphenyl-3'-{)2-methoxy)ethoxycarbonylmethoxycarbonyl}-4'-nitrophenyl ether

A suspension of 2-chloro-4-trifluoromethyl-3'-carboxy methoxycarbonyl-4'-nitrodiphenylether (7.1 g) in thionyl chloride (2.5 g) was heated for 1 hour at 110°C and then excess thionyl chloride was stripped off under reduced pressure. 2-Methoxyethanol (40 ml) was added and the mixture heated briefly to 95°C to form a solution. Excess 2-methoxyethanol was removed under reduced pressure and the residue taken up in ether and the solution washed with water and potassium carbonate solution, dried, filtered through activated silica gel, and stripped to yield the desired ester (7.5 g) as a yellow oil. Found: C, 48.40; H, 3.13; Cl, 8.01; F, 12.13; N, 3.62; $C_{19}H_{15}ClF_3NO_8$ requires C, 47.76; H, 3.16; Cl, 7.42; F, 11.93; N, 2.93.

Example 13

2-Chloro-4-trifluoromethyl-3'-(trifluoroethoxycarbonyl)-methoxycarbonyl-4'-nitrodiphenyl ether

This compound was prepared from 2-chloro-4-trifluoromethyl-3'-(carboxymethoxycarbonyl)-4'-nitrodiphenyl ether and trifluoroethanol ($CF_3CH_2OH$) following substantially the procedure of Example 12.

Example 14

2-Chloro-4-trifluoromethyl-3'-methoxycarbonylmethoxycarbonyl-4'-nitrodiphenyl ether

The potassium salt of 2-chloro-4-trifluoromethyl-3'-carboxy-4'-nitrodiphenyl ether was prepared by dissolving the acid in methanol and adding an equivalent amount of potassium hydroxide in methanol and evaporating the methanol. The potassium salt was dissolved in dry dimethyl formamide and methyl $\alpha$-bromoacetate (14.3 g; 0.09375 mole) was added. The reaction mixture was heated to 120°C for 4 hours after which it was added to dilute hydrochloric acid and extracted with hexane. The hexane solution was washed until neutral and then filtered through activated silica gel. The solvent was removed to afford 16 g of 2-chloro-4-trifluoromethyl-3'-carbomethoxymethoxycarbonyl-4'-nitrodiphenyl ether. m.p. 97°—98°C.

Example 15

2-Chloro-4-trifluoromethylphenyl-3'-(allyloxycarbonylmethoxycarbonyl)-4'-nitrophenyl ether

To a mixture of 2-chloro-4-trifluoromethylphenyl-3'-carboxymethoxycarbonyl-4'-nitrophenyl ether (10.5 g) and potassium carbonate (4.1 g) in dimethylsulfoxide (30 ml) was added allyl bromide (3.0 g) at 25°C. The temperature was increased to 32°C and a yellow mixture was formed. A sample tested on thin layer chromatography showed a single spot. The yellow mixture was diluted with 300 ml of water and extracted with 2 x 200 ml portions of ether. The ether extract was washed with 100 ml of aqueous 5% potassium carbonate solution. The ether solution was dried over anhydrous magnesium sulfate, filtered through activated silica gel and the ether removed to afford 10.3 g of yellow oil which solidified. The solid was recrystallized from hexane to afford substantially pure 2-chloro-4-trifluoromethylphenyl-3'-(allyloxycarbonylmethoxycarbonyl)-4-nitrophenyl ether, m.p. 51°—53°C. Found: C, 49.94; H, 2.86; Cl, 7.97; F, 12.49; N, 2.99; $C_{19}H_{13}ClF_3NO_4$ requires C, 49.63; H, 2.85; Cl, 7.71; F, 12.40; N, 3.05.

Example 16

2-Chloro-4-trifluoromethylphenyl-3'-{(N,N-dimethyl)-carbamoylmethoxycarbonyl}-4'-nitrophenyl ether

To a well stirred mixture of an aqueous solution of dimethylamine (40% aqueous; 20 ml) and diethyl ether (100 ml) there was added dropwise 2-chloro-4-trifluoromethylphenyl-3'-chlorocarbonylmethoxycarbonyl-4'-nitrophenyl ether (4 g in 50 ml diethyl ether). The aqueous phase became clear yellow. The reaction mixture was stirred for 20 minutes, the aqueous phase discarded and the yellow ether solution was washed with aqueous 5% sulfuric acid and then with an aqueous 5% potassium carbonate solution. The ether solution was dried over anhydrous magnesium sulfate and filtered through activated silica gel. A precipitate formed which, after standing and cooling, was collected. The

filtrate was stripped and the crystals which formed were collected and, combined with the first crop of crystals, resulted in 4.3 g of substantially pure 2-chloro-4-trifluoromethylphenyl-3-{(N,N-dimethyl)-carbamoylmethoxycarbonyl}-4-nitrophenyl ether, m.p. 108.5°—110°C. FOund: C, 48.43; H, 3.07; Cl, 8.29; F, 13.27; N, 6.22. $C_{18}H_{14}ClF_3N_2O_6$ requires C, 48.38; H, 3.16; Cl, 7.94; F, 12.76; N, 6.27.

Example 17

Sodium and potassium salts of 2-chloro-4-trifluoromethyl-3′-(carboxymethoxycarbonyl)-4′-nitrodiphenyl ether

(a) To a suspension of 2-chloro-4-trifluoromethyl-3′-(carboxymethoxycarbonyl)-4′-nitrodiphenyl ether (2.1 g) in water (1,000 ml) there was slowly added dropwise over a 90 minute period an aqueous solution of sodium hydroxide (9.5 ml fo 0.5 N diluted to 50 ml). The reaction mixture was stirred for an additional 90 minutes and then filtered to remove undissolved starting materials. The water was removed under high vacuum at 30°C—40°C to afford 1.0 g of 2 - chloro - 4 - trifluoromethyl - 3′ - (carboxymethoxycarbonyl) - 4′ - nitrodiphenyl ether, sodium salt.

(b) By following substantially the procedure described in part (a) above and by substituting for the sodium hydroxide recited therein an equal molar quantity of aqueous potassium hydroxide there is obtained 1.2 g of the potassium salt of 2 - chloro - 4 - trifluoromethyl - 3′ - (carboxymethoxycarbonyl) - 4′ - nitrodiphenyl ether.

Example 18

Lithium salt of 2-chloro-4-trifluoromethyl-3′-(carboxymethoxycarbonyl)-4′-nitrodiphenyl ether

To a solution of 2-chloro-4-trifluoromethyl-3′-(carboxymethoxycarbonyl)-4′-nitrodiphenyl ether (2.1 g; 0.005 mole) in diethyl ether (100 ml) under nitrogen was added *n*-butyl lithium solution in hexane (2.6 ml of a 1.9 M solution; 0.0049 mole). The solution turned clear red. After 30 minutes of stirring the solution darkened. The reaction mixture was stirred overnight at room temperature. The solvent was removed to afford 2.4 g of the lithium salt of 2 - chloro - 4 - trifluoromethyl - 3′ - (carboxymethoxycarbonyl) - 4′ - nitrodiphenyl ether as a crystalline brown solid.

Example 19

2-Chloro-4-trifluoromethyl-3′-{α-(methoxycarbonyl)-α-(benzyl)methoxycarbonyl}-4′-nitrodiphenyl ether

To a stirred mixture of 2-chloro-4-trifluoromethyl-3′-carboxy-4′-nitrodiphenyl ether (10.85 g; 0.03 mole) in methylethyl ketone (30 ml) was added potassium carbonate (8.3 g; 0.06 mole). An immediate reaction with gas evolution occurred leaving a paper-like mass. After warming for 15 minutes at 60°C the mixture was cooled and methyl α-bromo-α-benzyl acetate (7.3 g; 0.03 mole) was added. The emulsion was very thick and an additional 80 ml of methylethyl ketone was added. The reaction mixture was allowed to stand overnight and then stirring was continued. An additional 200 ml of methylethyl ketone was added and the reaction mixture was heated to 60°C for 3 hours. The reaction mixture was cooled and diluted with diethyl ether (200 ml). The ether solution was washed with water (200 ml) and then with aqueous 5% sulfuric acid and, finally, with aqueous 5% potassium carbonate. The ether solution was dried over molecular sieves. The ether solution was filtered through activated silica gel and the ether removed to afford 8.3 g of a yellow oil. Thin layer chromatography indicated a substantial amount of unreacted ester. The yellow oil was diluted with methylethyl ketone (70 ml) and an additional quantity of the nitrodiphenyl ether (7.2 g; 0.02 mole) and potassium carbonate (3.4 g—0.02 mole) was added and the mixture heated to reflux and the refluxing was continued overnight. The product was extracted with ether and the ether solution was washed with water, then with aqueous 5% sulfuric acid and, finally, with aqueous 5% potassium carbonate solution. The ether solution was dried over anhydrous magnesium sulfate, filtered through activated silica gel and the ether removed to afford 1.9 g of 2 - chloro - 4 - trifluoromethyl - 3′ - (α - (methoxycarbonyl) - α - (benzyl)ethoxycarbonyl} - 4′ - nitrodiphenyl ether.

Example 20

2-Chloro-4-trifluoromethyl-3′-{α-(methoxycarbonyl)-α-(phenyl)methoxycarbonyl}-4′-nitrodiphenyl ether

To a mixture of 2-chloro-4-trifluoromethyl-3′-carboxy-4′-nitrodiphenyl ether (7.23 g; 0.02 mole) in methylethyl ketone (25 ml) was added potassium carbonate (5.52 g; 0.04 mole). An immediate reaction occurred with gas evolution. An additional 100 ml of methylethyl ketone was added, followed by the addition of methyl α-bromophenyl acetate (4.58 g; 0.02 mole). The reaction mixture was stirred for 90 minutes and allowed to stand overnight at room temperature. Stirring was continued for an additional 4 hours at room temperature and then the reaction mixture was heated to 65°C for 3 hours. The reaction mixture was cooled and diethyl ether (300 ml) was added. The ether solution was washed with water, then with aqueous 5% sulfuric acid and, finally, with aqueous 5% potassium carbonate solution. The ether phase was dried over molecular sieves, filtered through activated silica gel and the ether removed to afford 8.5 g of 2 - chloro - 4 - trifluoromethyl - 3′ - {α - (methoxycarbonyl) - α -

(phenyl) - methoxycarbonyl} - 4' - nitrodiphenyl ether as a yellow solid. Recrystallization from isopropanol afforded 5.0 g of fluffy white crystals, m.p. 114°—117°C.

Example 21

2-Chloro-4-trifluoromethyl-3'-{(ethoxycarbonyl)methylthiocarbonyl}-4'-nitrodiphenyl ether

2-Chloro-4-trifluoromethyl-3'-carboxy-4'-nitrodiphenyl ether (12.6 g; 0.03 mole) and thionyl chloride (4.2 g; 2.25 moles) was stirred and heated in a bath at 100°C. The mixture melted and heating was continued at 100°C for an additional hour. An additional 2 g of thionyl chloride was added and the mixture heated for 30 additional minutes. The reaction mixture was allowed to stand overnight at room temperature and reheated for 2 hours. The excess thionyl chloride was removed under vacuum and chlorine using an oil bath at 100°C. The dark brown oil obtained was cooled and diluted with diethyl ether (50 ml). To this dark solution was added ethyl 2-mercaptoacetate (3.7 g). The reaction mixture was stirred for 2 hours at room temperature and then allowed to stand for 9 days (albeit fortuitously). The mixture was then diluted with diethyl ether and washed in water. The ether solution was washed with aqueous 5% sulfuric acid and then with aqueous 5% potassium carbonate. The ether solution was dried over anhydrous magnesium sulfate and then filtered through activated silica gel. Removal of the ether afforded 13.1 g of product. This material was dissolved in diethyl ether (43 ml) and enough hexane (85 ml) was added until the solution became hazy. The hazy solution was passed through activated silica gel and the solvent removed to afford 9.1 g of 2 - chloro - 4 - trifluoromethyl - 3' - {(ethoxycarbonyl)methylthiocarbonyl} - 4' - nitrodiphenyl ether as a red oil.

Example 22

2-Chloro-4-trifluoromethyl-3-{$\alpha$(ethoxycarbonyl)propoxycarbonyl}4'-nitrodiphenyl ether

A mixture of 2-chloro-4-trifluoromethyl-3'-carboxy-4'-nitrodiphenyl ether (10.9 g; 0.03 mole) and granular anhydrous potassium carbonate (8.3 g) in methylethyl ketone (50 ml) was heated for 1 hour at 60°—70°C and then cooled. Ethyl 2-bromobutyrate (5.9 g; 0.03 mole) was then added and the emulsion warmed to 50°C for 2 hours. The mixture was allowed to stand at room temperature overnight. Heating was resumed at 60°—70°C for an additional 4 hours. Ether (100 ml) was added and the ether solution washed with water, followed by washing with aqueous 5% sulfuric acid and, finally, with aqueous 5% potassium carbonate. The ether solution was dried over magnesium sulfate and then filtered through activated silica gel. The ether was removed to afford 6.2 g of 2 - chloro - 4 - trifluoromethyl - 3 - {$\alpha$(ethoxycarbonyl)propoxycarbonyl} - 4' - nitro - diphenyl ether. The material was dissolved in hexane (except for about 0.3 g of orange oil) and the hexane decanted and removed to afford 5.6 g of product. This material was redissolved in hexane and passed through 40 g of activated silica gel in hexane. Three cuts were taken and the last two combined and the hexane removed to afford 2.5 g of product as a yellow oil.

Example 23

2-Chloro-4-trifluoromethyl-3-{N-(1-ethoxycarbonylethyl)-carbamoyl}-4'-nitrodiphenyl ether

2-Chloro-4-trifluoromethyl-3'-carboxy-4'-nitrodiphenyl ether (0.04 mole) and thionyl chloride (5.4 g; 0.045 mole) were mixed together and refluxed for two hours. 100 ml of diethyl ether were added to the reaction mixture which was then added dropwise to a mixture of ethyl L-analine hydrochloride (6.1 g; 0.04 mole) and 2,6-lutidine (8.51 g; 0.08 mole) dissolved in 25 ml of diethyl ether. The resultant red and black solution was then stirred at room temperature overnight. The black solution was diluted with diethyl ether and then washed with water. The ether solution was then washed in aqueous 5% sulfuric acid followed by washing with aqueous 5% potassium carbonate. The ether solution was dried over anhydrous magnesium sulfate and filtered through activated silica gel. Removal of the ether afforded a black oil which soon solidified. The solid was dissolved in hot hexane. The hexane was decanted and allowed to cool. The residual oil was extracted with hexane and the hexane extracts placed in a refrigerator. The first hexane extract afforded 0.06 g of product having a melting point of 45°—47°C. The second extract afforded a red black solid glass-like material, 3.9 g having a melting point of 40°—49°C.

Example 24

2-Chloro-4-trifluoromethyl-3'-{$\alpha$-(ethoxycarbonyl)isobutoxycarbonyl}-4'-nitrodiphenyl ether

2-Chloro-4-trifluoromethyl-3'-carboxy-4'-nitrodiphenyl ether (14.5 g; 0.04 moles) was dissolved in dimethyl sulfoxide (40 ml). Potassium carbonate (11.0 g; 0.08 moles) was added to this mixture. The reaction mixture was then heated to 60°C. The reaction mixture was cooled to room temperature and ethyl 2-bromo-3-methylbutyrate (8.4 g; 0.04 moles) in dimethyl sulfoxide (10 ml) was then added. The reaction mixture was stirred at room temperature and allowed to stand and then extracted with diethyl ether, the ether solution being washed successively with water, aqueous 5% sulfuric acid solution and aqueous 5% potassium carbonate solution. The ether solution was dried over anhydrous magnesium sulfate and filtered through activated silica gel. Removal of the ether afforded 4.6 g of 2 - chloro - 4 - trifluoromethyl - 3' - {$\alpha$ - (ethoxycarbonyl) - isobutoxycarbonyl} - 4' - nitrodiphenyl ether.

Example 25

2-chloro-4-trifluoromethyl-3'-{1-(aminocarbonyl)ethoxycarbonyl}-4'-nitrodiphenyl ether

2-chloro-4-trifluoromethyl-3'-carboxy-4'-nitrodiphenyl ether (30.0 g) was dissolved in dimethyl sulfoxide (30 ml) and heated to 80°C. Potassium carbonate (12.6 g) was then added. Foaming occurred and, when the foaming ceased, the mixture was cooled in an ice bath to 38°C. $\alpha$-Bromopropionamide (13.9 g) was added and the reaction mixture was heated for $1\frac{1}{2}$ hours at 95°C. The reaction mixture was diluted with ether and the ether extract was washed successively with water, aqueous 1% sodium hydroxide and aqueous 5% sulfuric acid. The ether was removed and the residue crystallized from toluene. The toluene solution was filtered and crystals formed which were not the desired amine. The filtrate was stripped to afford 4.0 g of desired 2 - chloro - 4 - trifluoromethyl - 3' - {1 - (aminocarbonyl) - ethoxycarbonyl} - nitrodiphenyl ether.

Example 26

2-Chloro-4-trifluoromethyl-3'{1-(carboxy)ethoxycarbonyl}-4'-nitrodiphenyl ether

The 2 - chloro - 4 - trifluoromethyl - 3' - {1 - (methoxycarbonyl) - ethoxycarbonyl} - 4' - nitrodiphenyl ether of Example 5 (47.7 g; 0.107 moles) was mixed together with *p*-toluene-sulfonic acid (1.0 g), dioxane (180 ml) and water (5 ml). The reaction mixture was heated, with stirring, to reflux for 6 hours. The reaction mixture was diluted with aqueous 5% sulfuric acid and then extracted with ether. The ether extract was itself extracted with aqueous 1% sodium hydroxide. This base extract was acidified with aqueous 5% sulfuric acid and extracted with ether. The first ether extract was dried over anhydrous magnesium sulfate, filtered through activated silica gel and the ether removed to afford 12.3 g of 2 - chloro - 4 - trifluoromethyl - 3'{1 - (carboxy) - ethoxycarbonyl} - 4' - nitrodiphenyl ether.

Example 27

Sodium salt of 2-Chloro-4-trifluoromethyl-3'-{1-(carboxy)-ethoxycarbonyl}-4'-nitrodiphenyl ether

The acid of Example 32 (2.2 g) was dissolved in methanol (20 ml). To this solution was added sodium methoxide (0.27 g; 0.005 mole) dissolved in methanol (20 ml). The reaction mixture was stirred for 10 minutes and the methanol removed. The product was extracted with ether and the ether removed to afford 2.1 g of the sodium salt of 2-chloro-4-trifluoromethyl-3'{1-(carboxy)ethoxycarbonyl}-4'-nitrodiphenyl ether.

Example 28

Potassium salt of 2-Chloro-4-trifluoromethyl-3'-{1-(carboxy)-ethoxycarbonyl}-4'-nitrodiphenyl ether

The product of Example 32 (2.2 g) was dissolved in methanol. To this solution was added a solution of potassium hydroxide (0.28 g) in methanol (20 ml). The reaction mixture was stirred for 10 minutes and the methanol removed under vacuum. The product was extracted with ether and the ether removed to afford 2.1 g of 2 - chloro - 4 - trifluoromethyl - 3'{1 - (carboxy)ethoxycarbonyl} - 4' - nitrodiphenyl ether, potassium salt, m.p. 100°—105°C.

Example 29

2-Chloro-4-trifluoromethyl-3'-{$\beta$-(ethoxycarbonyl)-$\alpha$-methylethoxycarbonyl}-4'-nitrodiphenyl ether

A mixture of 2-chloro-4-trifluoromethyl-3'-chlorocarbonyl-4'-nitrodiphenyl ether (10 g); ethyl 3-hydroxybutyrate (3.5 g) and lutidine (2.8 g) in toluene (15 ml) was heated with stirring to 55°C. Heating was continued (in the event fortuitously) for two days. The reaction mixture was diluted with water and extracted with ether. The ether solution was washed with a 5% sodium carbonate solution and then water. The ether solution was then dried over magnesium sulfate filtered through charcoal and activated silica gel and the solvent removed to yield 8.2 g of 2 - chloro - 4 - trifluoromethyl - 3' - {$\beta$ - (ethoxycarbonyl) - $\alpha$ - methylethoxycarbonyl) - 4' - nitrodiphenyl ether as an oil.

Example 30

2-Chloro-4-trifluoromethyl-3'-($\omega$-ethoxycarbonyl)-n-butoxycarbonyl-4'-nitrodiphenyl ether

2-Chloro-4-trifluoromethyl-3'-carboxy-4'-nitrodiphenyl ether (10.85 g; 0.03 moles) was dissolved in methylethyl ketone (30 ml) and potassium carbonate (8.3 g; 0.06 moles) added; a solid formed. Additional methyl ethyl ketone (40 ml) was added and the emulsion heated in an oil bath at 100°C and then cooled in an ice bath. Ethyl 5-bromovalerate (6.27 g; 0.03 ml) was added and also additional methyl ethyl ketone (10 ml). The reaction mixture was heated to 60°C for 5 minutes, cooled and stirred at room temperature for 2 hours at room temperature. The mixture was colored pink-purple. The emulsion was diluted with ether and the ether solution washed with water, then with aqueous 5% sulfuric acid and finally with aqueous 5% potassium carbonate. The ether solution was dried over anhydrous magnesium sulfate and filtered through activated silica gel. The ether was removed to afford 8.0 g of yellow oil. NMR showed impurity of ethyl 5-bromovalerate which was removed by distillation. The residue was 2.5 g of 2 - chloro - 4 - trifluoromethyl - 3' - ($\omega$ - ethoxycarbonyl) - n - butoxycarbonyl) - 4' - nitrodiphenyl ether. NMR and IR confirmed the identity of the product.

Example 31

2-Chloro-4-trifluoromethyl-3'-ethoxycarbonylmethoxycarbonyl-4'-nitrodiphenyl ether

2-Chloro-4-trifluoromethyl-3'-carboxy-4'-nitrodiphenylether (193 g) was dissolved in methyl ethyl ketone (600 ml) and the mixture heated to reflux. Anhydrous potassium carbonate (81 g) was added portionwise with stirring over one hour. The temperature was then reduced to 72°C and ethyl bromoacetate (100 g) was added and the reaction mixture heated at 60—70°C for about 20 hours. The reaction mixture was diluted with water and extracted with ether. The ether phase was washed with 5% aqueous potassium carbonate solution, and brine, dried over anhydrous sodium sulphate, filtered through activated silica gel and the solvent removed to give 2 - chloro - 4 - trifluoromethyl - 3' - ethoxycarbonylmethoxycarbonyl - 4' - nitrodiphenylether m.p. 44—52°C.

TABLE II
DIPHENYL ETHERS — PHYSICAL DATA

| Example No. | m.p | Molecular Formula | C found (reqd) | H found (reqd) | N found (reqd) | Cl found (reqd) |
|---|---|---|---|---|---|---|
| 1 | 25°C | $C_{20}H_{17}Cl_1F_3N_1O_7$ | 50.13(50.49) | 3.51 (3.60) | 2.87(2.94) | 7.38(7.45) |
| 2 | 103.5—106°C | $C_{16}H_7Cl_1F_3N_1O_7$ | 45.99(45.79) | 2.59(2.16) | 2.88(3.34) | 8.00(8.45) |
| 3 | — * | $C_{18}H_{13}Cl_1F_3N_1O_7$ | 46.94(48.29) | 2.90(2.93) | 2.93(3.13) | 8.59(7.92) |
| 4 | — * | $C_{19}H_{15}Cl_1F_3N_1O_7$ | 49.16(49.39) | 3.19(3.27) | 3.31(3.03) | 8.20(7.68) |
| 5 | 70—71.5°C | $C_{18}H_{16}Cl_1F_3N_2O_5$ | 48.94(49.95) | 3.74(3.73) | 6.18(6.47) | 8.14(8.19) |
| 9 | — * | $C_{20}H_{17}Cl_1F_3N_1O_7$ | 50.66(50.49) | 3.80(3.60) | 3.02(2.94) | 8.27(7.45) |
| 11 | 92—94.5°C | $C_{20}H_{17}Cl_1F_3N_1O_7$ | 50.15(50.45) | 3.54(3.60) | 2.89(2.94) | 7.54(7.45) |
| 12 | — * | $C_{19}H_{15}Cl_1F_3N_1O_8$ | 48.40(47.76) | 3.13(3.16) | 3.62(2.93) | 8.01(7.42) |
| 13 | — * | $C_{18}H_{10}Cl_1F_6N_1O_7$ | 43.78(43.09) | 2.02(2.01) | 2.79(2.79) | 7.04(7.07) |
| 14 | 97—98°C | $C_{17}H_{11}Cl_1F_3N_1O_7$ | 45.36(47.07) | 2.76(2.56) | 3.26(3.23) | 9.26(8.17) |
| 15 | 51—53°C | $C_{19}H_{13}Cl_1F_3N_1O_4$ | 49.94(49.63) | 2.86(2.85) | 2.99(3.05) | 7.97(7.71) |
| 16 | 108.5—110°C | $C_{18}H_{14}Cl_1F_3N_2O_6$ | 48.43(48.38) | 3.07(3.16) | 6.22(6.27) | 8.29(7.94) |
| 17(a) | — | $C_{16}H_8Cl_1F_3N_1O_7Na$ | 41.72(43.51) | 1.91(1.83) | 2.82(3.17) | 5.32(8.03) |
| 17(b) | — | $C_{16}H_8Cl_1F_3N_1O_2K_1$ | 45.15(41.98) | 2.04(1.76) | 3.19(3.06) | 8.66(7.74) |
| 18 | — | $C_{16}H_8Cl_1F_3N_1O_7Li$ | 45.65(45.15) | 2.88(1.89) | 2.80(3.29) | 7.99(8.33) |
| 19 | — * | $C_{24}H_{17}Cl_1F_3N_1O_7$ | 55.29(55.02) | 3.41(3.27) | 2.83(2.67) | 7.27(6.77) |
| 20 | 114—117°C | $C_{23}H_{15}Cl_1F_3N_1O_7$ | 54.28(54.18) | 2.83(2.96) | 2.94(2.75) | 7.02(6.96) |
| 21 | — * | $C_{18}H_{13}Cl_1F_3N_1O_6S_1$ | 46.33(46.60) | 2.72(2.82) | 2.98(3.02) | 8.43(7.44) |
| 22 | — * | $C_{20}H_{17}Cl_1F_3N_1O_7$ | 52.28(50.48) | 3.85(3.60) | 3.01(2.94) | 7.65(7.45) |
| 23 | 45—47°C | $C_{19}H_{16}Cl_1F_3N_2O_6$ | 49.08(49.52) | 3.06(3.50) | 5.24(6.08) | 1.89(7.70) |
| 24 | — * | $C_{21}H_{19}Cl_1F_3N_1O_7$ | 50.40(51.45) | 3.82(3.91) | 3.14(2.86) | 9.16(7.24) |
| 25 | — * | $C_{17}H_{12}Cl_1F_3N_2O_6$ | 51.67(47.18) | 3.46(2.80) | 5.58(6.47) | 8.07(8.19) |
| 26 | — * | $C_{17}H_{11}Cl_1F_3N_1O_7$ | 47.23(47.08) | 2.87(2.56) | 2.88(3.23) | 7.59(8.17) |
| 27 | 105—110°C | $C_{17}H_{11}Cl_1F_3N_1O_2Na$ | 44.27(44.81) | 2.14(2.21) | 3.29(3.07) | 7.42(7.78) |
| 28 | 100—105°C | $C_{17}H_{11}Cl_1F_3N_1O_7K$ | 43.88(43.28) | 2.59(2.14) | 2.88(2.97) | 7.43(7.51) |
| 29 | — * | $C_{20}H_{17}Cl_1F_3N_1O_7$ | 53.96(50.49) | 3.99(3.60) | 3.05(2.94) | 7.23(7.45) |
| 30 | — * | $C_{21}H_{19}Cl_1F_3N_1O_7$ | 53.46(51.49) | 4.16(3.91) | 2.71(2.86) | 7.38(7.24) |
| 31 | 44—52°C | $C_{18}H_{13}Cl_1F_3N_1O_7$ | 48.28(48.30) | 2.94(2.90) | 3.01(3.10) | 8.07(7.90) |

* = oil

Table IIA which follows gives NMR data for the oils in Table II.

TABLE IIA

| Example No. | N.M.R. Data — Oils |
|---|---|
| 3 | $^1$H n.m.r. δ($CDCl_3$) 8.1—6.9 (m, 6H), 5.4 (quart., 1H), 3.8 (s, 3H), 1.6 (d, 3H). |
| 4 | $^1$H n.m.r. δ($CDCl_3$) 8.2—6.7 (m, 6H), 5.4 (quart., 1H), 4.2 (quart., 2H), 1.6 (d, 3H), 1.3 (t, 3H). |
| 9 | $^1$H n.m.r. δ($CDCl_3$) 8.1—7.1 (m, 6H), 4.3 (t, 2H), 4.0 (quart., 2H), 2.6—1.8 (m, 4H), 1.1 (t, 3H). |
| 12 | $^1$H n.m.r. δ($CDCl_3$) 8.2—7.0 (m, 6H), 4.9 (s, 2H), 4.5—4.3 (m, 2H), 3.7—3.5 (m, 2H), 3.4 (s, 3H). |
| 13 | $^1$H n.m.r. δ($CDCl_3$) 8.1—6.9 (m, 6H), 4.9 (s, 2H), 4.5 (quart., 2H). |
| 19 | $^1$H n.m.r. δ($CDCl_3$) 8.0—6.9 (m, 11H), 5.5 (t, 1H), 3.7 (d, 2H), 3.6 (s, 3H). |
| 21 | $^1$H n.m.r. δ($CDCl_3$) 8.3—7.0 (m, 6H), 4.2 (quart., 2H), 3.9 (s, 2H), 1.2 (t, 3H). |
| 22 | $^1$H n.m.r. δ($CDCl_3$) 8.2—7.0 (m, 6H), 5.2 (t, 1H), 4.2 (quart., 2H), 2.3—1.7 (m, 2H), 1.4—.8 (m, 6H). |
| 24 | $^1$H n.m.r. δ($CDCl_3$) 8.8—6.9 (m, 6H), 5.0 (d, 1H), 4.2 (quart., 2H), 2.6—1.9 (m, 1H), 1.4—.9 (m, 9H). |
| 25 | $^1$H n.m.r. δ($CDCl_3$) 8.2—7.0 (m, 6H), 6.4 (s, 2H), 5.5 (quart., 1H), 1.6 (d, 3H). |
| 26 | $^1$H n.m.r. δ($CDCl_3$) 10.3 (s, 1H), 8.1—6.9 (m, 6H), 5.4 (quart., 1H), 1.6 (d, 3H). |
| 29 | $^1$H n.m.r. δ($CDCl_3$) 8.0—6.8 (m, 6H), 5.5 (quart., 1H), 4.1 (quart., 2H), 2.6 (d, 2H), 1.3 (t, 3H), 1.2 (t, 3H). |
| 30 | $^1$H n.m.r. δ($CDCl_3$) 8.1—6.9 (m, 6H), 4.5—4.2 (m, 2H), 4.2 (quart., 2H), 2.6—2.2 (m, 2H), 2.0—1.6 (m, 2H), 1.2 (t, 3H). |

It will be evident that compounds of Formula I may be used in the preparation of other compounds of Formula I. For example, it will be seen from some of the foregoing examples that there may be used for this purpose compounds of Formula I wherein Y is oxygen and Z is carboxy, a carboxy ester, a carboxy salt or a carboxy amide (hereinafter for brevity referred to as "precursor compounds").

An example (*vide* Example 12) of the use of such a precursor compound is the conversion of a free carboxylic acid (i.e. Z = COOH) to an acid chloride, fluoride or bromide, e.g. by reaction with a halogenating agent such as thionyl halide in a suitable liquid medium. The acid halide can then be reacted with ammonia or an amine (e.g. mono- or di-($C_1$—$C_4$)-alkyl amines) to give an amide. Alternatively (*vide* Examples 12 and 13), the acid halides may be reacted with an appropriate hydroxy compound, if necessary in the presence of a hydrogen halide acceptor, to give an ester. Examples of hydroxy compounds are alkanols optionally substituted with halo, alkoxy and/or haloalkoxy.

The precursor compounds of Formula I wherein Z is carboxy may be converted to salts, e.g. by reaction in solution or suspension with a strong base such as an alkali metal hydroxide (cf. Examples 17 and 18 or an alkaline earth metal hydroxide. The salts can then be reacted with an appropriate chloro, bromo or fluoro compound to give an ester, an example of this reaction being given in Example 15.

When the precursor compounds are esters of compounds of Formula I wherein Z is COOH, the esters may be converted by hydrolysis to the free acids as in Example 26. In some cases the esters may converted to the free acids simply by thermal decomposition as in Example 2 where the *t*-butyl ester is thermally decomposed by the loss of isobutylene.

The following is a listing of some of the preferred compounds of Formula I.

TABLE III

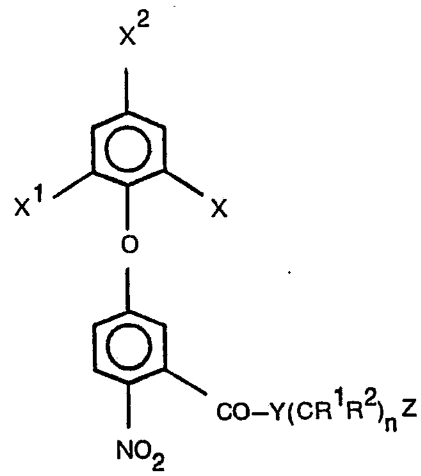

| Example | X | $X^1$ | $X^2$ | Y | $R^1$ | $R^2$ | n | Z |
|---|---|---|---|---|---|---|---|---|
| 1 | Cl | H | $CF_3$ | O | H | H | 1 | $CO_2$-*t*-$C_4H_9$ |
| 2 | Cl | H | $CF_3$ | O | H | H | 1 | $CO_2H$ |
| 3 | Cl | H | $CF_3$ | O | $CH_3$ | H | 1 | $CO_2CH_3$ |
| 4 | Cl | H | $CF_3$ | O | $CH_3$ | H | 1 | $CO_2C_2H_5$ |
| 9 | Cl | H | $CF_3$ | O | H | H | 3 | $CO_2C_2H_5$ |
| 11 | Cl | H | $CF_3$ | O | $CH_3$ | $CH_3$ | 1 | $CO_2C_2H_5$ |
| 12 | Cl | H | $CF_3$ | O | H | H | 1 | $CO_2CH_2CH_2OCH_3$ |
| 13 | Cl | H | $CF_3$ | O | H | H | 1 | $CO_2CH_2CF_3$ |
| 14 | Cl | H | $CF_3$ | O | H | H | 1 | $CO_2CH_3$ |
| 15 | Cl | H | $CF_3$ | O | H | H | 1 | $CO_2CH_2CH{=}CH_2$ |
| 16 | Cl | H | $CF_3$ | O | H | H | 1 | $CON_3(CH_3)_2$ |
| 17 | Cl | H | $CF_3$ | O | H | H | 1 | $CO_2Na$ |
| 17a | Cl | H | $CF_3$ | O | H | H | 1 | $CO_2K$ |
| 18 | Cl | H | $CF_3$ | O | H | H | 1 | $CO_2Li$ |
| 19 | Cl | H | $CF_3$ | O | $CH_2C_6H_5$ | H | 1 | $CO_2CH_3$ |
| 20 | Cl | H | $CF_3$ | O | $C_6H_5$ | H | 1 | $CO_2CH_3$ |
| 21 | Cl | H | $CF_3$ | S | H | H | 1 | $CO_2C_2H_5$ |
| 22 | Cl | H | $CF_3$ | O | $C_2H_5$ | H | 1 | $CO_2C_2H_5$ |
| 23 | Cl | H | $CF_3$ | NH | $CH_3$ | H | 1 | $CO_2C_2H_5$ |
| 24 | Cl | H | $CF_3$ | O | i-$C_3H_7$ | H | 1 | $CO_2C_2H_5$ |
| 25 | Cl | H | $CF_3$ | O | $CH_3$ | H | 1 | $CONH_2$ |

TABLE III (continued)

| Example | X | $X^1$ | $X^2$ | Y | $R^1$ | $R^2$ | n | Z |
|---|---|---|---|---|---|---|---|---|
| 26 | Cl | H | $CF_3$ | O | $CH_3$ | H | 1 | $CO_2H$ |
| 27 | Cl | H | $CF_3$ | O | $CH_3$ | H | 1 | $CO_2Na$ |
| 28 | Cl | H | $CF_3$ | O | $CH_3$ | H | 1 | $CO_2K$ |
| 29 | Cl | H | $CF_3$ | O | $CH_3$(H)* | H | 2 | $CO_2C_2H_5$ |
| 30 | Cl | H | $CF_3$ | O | H | H | 4 | $CO_2C_2H_5$ |
| 31 | Cl | H | $CF_3$ | O | H | H | 1 | $CO_2C_2H_5$ |

*i.e. $-(CR^1R^2)_n- = -CH(CH_3)CH_2-$

Additional compounds of interest — and which form part of Table III above — are those in which (a) the $-(CR^1R^2)_n-$ group shown for each of the compounds of Examples 1—28 and 30 is replaced by each and every group selected from the following: $-CH_2CH_2-$, $-CH_2CH(CH_3)-$, $-CH_2C(CH_3)_2-$, $-CH(CH_3)CH_2-$, $-CH(CH_3)CH(CH_3)-$, $-CH(CH_3)C(CH_3)_2-$, $-C(CH_3)_2CH_2-$, $-C(CH_3)_2CH(CH_3)$ and $-C(CH_3)_2C(CH_3)_2-$ and (b) the $-(CR^1R^2)_n-$ group shown for the compound of Example 29 is replaced by each and every one of the following groups, viz $-CH_2CH_2-$, $-CH_2CH(CH_3)-$, $-CH_2C(CH_3)_2-$, $-CH(CH_3)CH(CH_3)-$. $-CH(CH_3)C(CH_3)_2-$, $-C(CH_3)_2CH_2-$, $-C(CH_3)_2CH(CH_3)-$ and $-C(CH_3)_2C(CH_3)_2-$. Yet further additional compounds of interest — and which also form part of Table III — are those in which (a) X = Cl in each of the compounds of Examples 1—30 is replaced by each and every one of the following substituents viz fluorine, bromine, trifluoromethyl, trichloromethyl, methyl, n-butyl, nitro and cyano, (b) $X^1$ = H in each of the compounds of Examples 1—30 is replaced by each and every one of chlorine, fluorine, bromine, trifluoromethyl and trichloromethyl and (c) $X^2 = CF_3$ in each of the compounds of Examples 1—30 is replaced by each of trichloromethyl and tribromomethyl. Specific examples of additional compounds are those wherein the $-CH(CH_3)-$ group in the compound of Example 3 is replaced by $-(CH_2)_2-$ or $-CH(CH_3)CH_2-$ and wherein the $-CH(CH_3)-$ group in the compound of Example 4 is replaced by $-C(CH_3)_2CH_2-$, $-CH(CH_3)CH(CH_3)-$ or $-(CH_2)_2-$.

*Herbicidal Activity*

Many of the diphenyl ethers of Formula I were evaluated on the following representative species:

| | | Approximate No. Seeds. |
|---|---|---|
| *Monocots:* | Barnyardgrass (*Echinochloa crusgalli*) | 25 |
| | Downybrome (*Bromus tectorum*) | 20 |
| | Foxtail (*Setaria* spp) | 25 |
| | Johnsongrass (*Sorghum halepense*) | 25 |
| | Nutsedge (*Cyperus esculentus*) | 5 |
| | Wild Oat (*Avena fatua*) | 20 |
| *Dicots:* | Cocklebur (*Xanthium pensylvanicum*) | 3 |
| | Marigold (*Tagetes* spp) | 15 |
| | Morning-glory (*Ipomoea* spp) | 10 |
| | Tomato (*Lycopersicon esculentum*) | 15 |
| | Velvetleaf (*Abutilon theophrasti*) | 15 |

The following test procedure was employed. Seeds of the above species were planted in soil in trays (approximately 18 cm x 27 cm x 8 cm). For preemergence tests, the trays were sprayed with the test compound immediately after planting. For postemergence tests, the seeds were allowed to germinate and after growing in the greenhouse for two weeks, the growing plants were treated with the test compound. The compound to be evaluated was dissolved or dispersed in acetone or water and sprayed over the trays using a carrier volume equivalent to 468 l/ha (50 U.S. gallons per acre (A)) at the rate of application (in kg/ha) specified in the table. About two weeks after application of the test compound, the state of growth of the plants was observed and the phytotoxic effect of each compound determined as follows: each species was evaluated on a scale of 0—100 in which 0 = no activity and 100 = total kill and the results for the monocots and dicots separately averaged. The following table shows the results obtianed for the compounds of the invention at 0.56 kg/ha (0.5 lb/A) and 2.24 kg/ha (2.0 lb/A) *exept as noted**:

Diphenylethers — Herbicidal Activity

| Example No. | Rate 0.56 kg/ha | | | | Rate 2.24 kg/ha | | | |
|---|---|---|---|---|---|---|---|---|
| | PRE | | POST | | PRE | | POST | |
| | AM+ | AD+ | AM | AD | AM | AD | AM | AD |
| 1 | 42 | 52 | 10 | 44 | 77 | 98 | 23 | 55 |
| 2 | 62 | 58 | 38 | 93 | 93 | 98 | 57 | 96 |
| 3 | 48 | 39 | 60 | 92 | 76 | 99 | 68 | 98 |
| 4 | 45 | 58 | 43 | 92 | 83 | 100 | 50 | 98 |
| 5 | — | — | — | — | 0 | 0 | 32 | 86 |
| 9 | 31 | 85 | 37 | 98 | 56 | 100 | 75 | 100 |
| 11 | 42 | 76 | 18 | 80 | 75 | 97 | 25 | 96 |
| 12 | 15 | 38 | 3 | 79 | 70 | 94 | 37 | 100 |
| *13 | 73 | 77 | 43 | 99 | 72 | 92 | 46 | 98 |
| 14 | 55 | 55 | 52 | 100 | 94 | 99 | 75 | 100 |
| 15 | 27 | 44 | 10 | 84 | 90 | 98 | 25 | 95 |
| 16 | 25 | 44 | 2 | 54 | 58 | 82 | 17 | 72 |
| 17a | 38 | 46 | 25 | 89 | 88 | 89 | 70 | 100 |
| *17b | 74 | 88 | 53 | 96 | 87 | 99 | 40 | 100 |
| *18 | 76 | 91 | 27 | 96 | 74 | 97 | 31 | 91 |
| 19 | 18 | 59 | 5 | 38 | 68 | 72 | 10 | 51 |
| 20 | 34 | 76 | 0 | 24 | 23 | 57 | 0 | 18 |
| 21 | 42 | 36 | 7 | 78 | 78 | 93 | 30 | 94 |
| 22 | 60 | 99 | 42 | 99 | 75 | 100 | 67 | 100 |
| 23 | 13 | 40 | 0 | 78 | 50 | 54 | 0 | 79 |
| 24 | 22 | 92 | 3 | 59 | 40 | 100 | 12 | 79 |
| 25 | 23 | 75 | 15 | 66 | 66 | 96 | 15 | 94 |
| *26 | 79 | 84 | 28 | 99 | | | | |
| *27 | 64 | 48 | 20 | 98 | | | | |
| *28 | 62 | 58 | 22 | 100 | | | | |
| 29 | 45 | 74 | 35 | 96 | 91 | 99 | 64 | 99 |
| 30 | 58 | 72 | 32 | 98 | 40 | 82 | 58 | 95 |
| 31 | 48 | 66 | 34 | 95 | 86 | 100 | 58 | 99 |

* 1.12 kg/ha (1.0 lb/A)
+AM = Average Monocot; AD = Average Dicot

The diphenyl ethers of Formula I are useful as preemergence and postemergence herbicides. Preemergence herbicides are ordinarily used to treat the soil by application either before seeding, during seeding, or after seeding with the crop and before the crop emerges. Postemergence herbicides are those which are applied after the crop plants have emerged and during their growth period. The compounds of Formula I are especially active as postemergence herbicides. Thus the invention provides a method of combating weeds, usually in an agronomic crop area, which comprises applying to the growing weeds, e.g. weed seedlings, or to the surface of a growth medium prior to emergence of the weeds, one or more of the diphenyl ethers in an amount sufficient to combat the growth of the weeds.

Among the crops on which the diphenyl ethers of the invention can be advantageously employed are, cotton, soybeans, peanuts, beans, peas, carrots, maize, wheat and other cereal crops.

The diphenyl ethers of Formula I are useful for controlling weeds in rice crops. When used in transplanted rice crops, the ethers can be applied either preemergence or postemergence to the weeds — that is, they can be applied to the transplanted rice plants and their growth medium either before the weed plants have emerged or while they are in their early stages of growth. The ethers can be applied to the growth medium either before or after the rice has been transplanted to that medium.

The diphenyl ethers of Formula I can be applied in any amount which will give the required control of weeds. A standard rate of application of the herbicides of the invention is in the range from about 0.022 to about 13.5 kg/ha (0.02 to 12 lb/A). A preferred range is from about 0.1 to about 2.24 kg/ha (0.1 to 2 lb/A).

Under some conditions, the diphenyl ethers may be advantageously incorporated into the soil or other growth medium prior to planting a crop. This incorporation can be by any convenient means, including simple mixing with the soil, applying the diphenyl ether to the surface of the soil and then discing or dragging into the soil to the desired depth or by employing a liquid carrier.

In their application as herbicides one or more of the compounds of Formula I will usually be used in conjunction with an agronomically acceptable carrier or diluent (and optionally a surfactant) so as to provide a herbicidal composition which, in general, will contain from 0.1 to 99.9% more usually 1 to 99% and even more usually 5 to 95% by weight of active herbicidal component comprising or consisting of one or more of the diphenyl ethers of Formula I. These herbicidal compositions may be in the form of ready-for-use dusts, solutions, emulsions, suspensions, granules or pellets; alternatively they may be in the form of concentrated solutions, wettable powders, water-dispersible granules, emulsifiable concentrates, flowable liquid suspensions, flowable emulsion concentrates, pastes or dust concentrates each for dilution with, as the case may be, additional liquid (usually water) or finely divided solids to give diluted solutions, suspensions, emulsions, or dusts which are then directly usable as herbicidal compositions.

By the term "agronomically acceptable carrier or diluent" as used above is meant a substance which can be utilized to dissolve, disperse, diffuse or otherwise dilute the active ingredient without impairing the effectiveness of the active ingredient and which does no permanent damage to such loci as soil, equipment and agronomic crops.

It is usually desirable, particularly in post-emergence applications, to include adjuvants, such as wetting agents, spreading agents, dispersing agents, stickers, adhesives, and the like, in accordance with agricultural practices. Examples of adjuvants which are commonly used in the art can be found in the John W. McCutcheon, Inc. publication "Detergents and Emulsifiers 1969 Annual".

Examples of solvents which are useful in the practice of this invention include alcohols, ketones, aromatic hydrocarbons, dimethyl formamide, dioxane, dimethyl sulfoxide, and the like. Mixtures of these solvents can also be used. Typical solutions contain about 2% to about 98% by weight of active ingredient and more usually about 25% to about 75% by weight.

For the preparation of emulsifiable concentrates, the diphenyl ether can be dissolved in organic solvents, such as benzene, toluene, xylene, methylated naphthalene, corn oil, pine oil, *o*-dichlorobenzene, isophorone, cyclohexanone, or methyl oleate, or in mixtures of these solvents, together with an emulsifying agent which permits dispersion in water. Suitable emulsifiers include, ethylene oxide derivatives of alkylphenols or long-chain alcohols, mercaptans, carboxylic acids, and reactive amines and partially esterified polyhydric alcohols. Solvent-soluble sulfates or sulfonates, such as the alkaline earth salts or amine salts of alkylbenzene-sulfonates and the fatty alcohol sodium sulfates, having surface-active properties can be used as emulsifiers either alone or in conjunction with an ethylene oxide reaction product. Flowable emulsion concentrates are formulated similarly to the emulsifiable concentrates and include, in addition to the above components, water and a stabilizing agent such as a water-soluble cellulose derivative or a water-soluble salt of a polyacrylic acid. Emulsifiable concentrates typically contain about 10% to 60% by weight of active ingredient and flowable emulsion concentrates can contain up to 75% by weight of active ingredient.

Wettable powders suitable for spraying, can be prepared by admixing the compound with a finely divided solid, such as clays, inorganic silicates and carbonates, and silicas and then incorporating wetting agents, sticking agents, and/or dispersing agents. The concentration of active ingredients in such formulations is typically in the range of from about 20% to about 98% by weight, and, preferably, from about 40% to about 75% by weight. A dispersing agent can constitute about 0.5% to about 3% by

weight of the composition, and a wetting agent can constitute from about 0.1% to about 5% by weight of the composition.

Dusts can be prepared by mixing the diphenyl ethers with finely divided inert solids which may be organic or inorganic in nature. Materials useful for this purpose include, for example, botanical flours, silicas, silicates, carbonates and clays. One convenient method of preparing a dust is to dilute a wettable powder with a finely divided carrier. Dust concentrates containing from about 20% to about 80% by weight of the active ingredient are commonly made and are subsequently diluted to about 1% to about 10% by weight use concentration.

Granular formulations can be prepared by impregnating a solid, such as granular fuller's earth, vermiculite, ground corn cobs, seed hulls, including bran or other grain-hulls, or similar material. A solution of one or more of the diphenyl ethers in a volatile organic solvent can be sprayed or mixed with the granular solid and the solvent then removed by evaporation. The granular material can have any suitable size, with a preferably size range of from 16 to 60 mesh (U.S. Standard Sieve Series) and typically contain from about 2 to about 15% by weight of active ingredient.

The diphenyl ethers of Formula I can also be mixed with fertilizers or fertilizing materials before their application. In one type of solid fertilizing composition in which the diphenyl ethers can be used, particles of a fertilizer or fertilizing ingredients, such as ammonium sulfate, ammonium nitrate, or ammonium phosphate, can be coated with one or more of the ethers. The solid diphenyl ethers and solid fertilizing material can also be admixed in mixing or blending equipment, or they can be incorporated with fertilizers in granular formulations. Any relative proportion of diphenyl ether and fertilizer can be used which is suitable for the crops and weeds to be treated. The diphenyl ether will commonly be present in an amount from about 5% to about 25% by weight of the fertilizing composition. These compositions provide fertilizing materials which promote the rapid growth of desired plants, and at the same time control the growth of undesired plants.

The diphenyl ethers can be applied as herbicidal sprays by methods commonly employed, such as conventional high-gallonage hydraulic sprays, low-gallonage sprays, airblast spray, aerial sprays and dusts. For low volume applications a solution of the compound is usually used. The dilution and rate of application will usually depend upon such factors as the type of equipment employed, the method of application, the area to be treated and the type and stage of development of the weeds.

For some applications, it may be desirable to add one or more other herbicides along with diphenyl ethers of the invention.

In addition to the numerous individual compounds already identified in the foregoing description there may, by way of completeness of disclosure, also be listed the following additional compounds provided by the invention.

SUPPLEMENTARY EXEMPLARY LIST

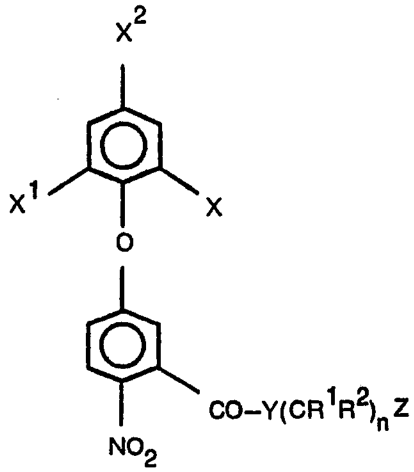

| Compound No. | X | $X^1$ | $X^2$ | Y | $R^1$ | $R^2$ | n | Z |
|---|---|---|---|---|---|---|---|---|
| 1 | Br | Cl | $CF_3$ | O | H | H | 1 | $CO_2C_2H_5$ |
| 2 | I | H | $CCl_3$ | O | H | H | 1 | $CO_2CH_3$ |
| 3 | *n*-$C_4H_9$ | Cl | $CF_3$ | O | H | H | 1 | $CON(CH_3)_2$ |
| 4 | *n*-$C_6H_{13}$ | Cl | $CF_3$ | O | H | H | 1 | $CO_2CH_3$ |
| 5 | *n*-$C_{12}H_{25}$ | H | $CF_3$ | O | H | H | 1 | $CO_2C_2H_5$ |
| 6 | Cl | Br | $CF_3$ | O | H | H | 1 | $CO_2C_2H_5$ |
| 7 | Cl | F | $CF_3$ | O | H | H | 1 | $CO_2C_2H_5$ |
| 8 | Cl | H | $CF_3$ | $NC_6H_5$ | H | H | 1 | $CO_2C_2H_5$ |
| 9 | Cl | H | $CF_3$ | $NCH_2C_6H_5$ | H | H | 1 | $CO_2CH_3$ |
| 10 | Cl | H | $CF_3$ | $NCH_3$ | H | H | 1 | $CO_2CH_3$ |
| 11 | Cl | H | $CF_3$ | N-*n*-$C_4H_9$ | H | H | 1 | $CO_2C_2H_5$ |
| 12 | Cl | H | $CF_3$ | O | H | H | 1 | Mg salt of $CO_2H$ |
| 13 | Cl | H | $CF_3$ | O | $CH_3$ | $CH_3$ | 1 | Ca salt of $CO_2H$ |
| 14 | Cl | H | $CF_3$ | O | H | H | 1 | $CO_2$-*n*-$C_6H_{17}$ |
| 15 | Cl | H | $CF_3$ | O | H | H | 1 | $CO_2(CH_2)_5CCl_3$ |
| 16 | Cl | H | $CF_3$ | O | H | H | 1 | $CO_2(CH_2)_5CF_3$ |
| 17 | Cl | H | $CF_3$ | O | H | H | 1 | $CO_2CH_2OCH_3$ |
| 18 | Cl | H | $CF_3$ | O | H | H | 1 | $CO_2(CH_2)_6O(CH_2)_3CH_3$ |
| 19 | Cl | H | $CF_3$ | O | H | H | 1 | $CO_2CH_2OCF_3$ |
| 20 | Cl | H | $CF_3$ | O | H | H | 1 | $CO_2(CH_2)_6O(CH_2)_3CCl_3$ |
| 21 | Cl | H | $CF_3$ | O | H | H | 1 | $CON(n\text{-}C_4H_9)_2$ |
| 22 | Cl | H | $CF_3$ | O | H | H | 1 | $CO_2CH_2CH{=}CH(CH_2)_2CH_3$ |

SUPPLEMENTARY EXEMPLARY LIST (continued)

| Compound No. | X | $X^1$ | $X^2$ | Y | $R^1$ | $R^2$ | n | Z |
|---|---|---|---|---|---|---|---|---|
| 23 | Cl | H | $CF_3$ | O | H | H | 1 | $CO_2CH_2{\equiv}CCH_3$ |
| 24 | Cl | H | $CF_3$ | O | H | H | 1 | $CO_2CH_2{\equiv}C(CH_2)_2CH_3$ |
| 25 | Cl | H | $CF_3$ | O | H | H | 1 | $NHCH_3$ |
| 26 | Cl | H | $CF_3$ | O | H | H | 1 | NH-*n*-$C_4H_9$ |
| 27 | Cl | H | $CF_3$ | O | H | H | 1 | $N(CH_3)_2$ |
| 28 | Cl | H | $CF_3$ | O | H | H | 1 | N(*n*-$C_4H_9)_2$ |
| 29 | Cl | H | $CF_3$ | O | H | H | 1 | $NH_2$ |

There should also be mentioned as specific compounds within the scope of Formula I all the individual diphenyl ethers of Formula I hereinbefore disclosed but in which the $-(CR^1R^2)_n-$ group of the individual diphenyl ether in question is replaced by a different group selected from each and every one of the following groups, viz $-CH_2CH_2-$, $-CH_2CH(CH_3)-$, $-CH_2C(CH_3)_2-$, $-CH(CH_3)CH_2-$, $-CH(CH_3)CH(CH_3)-$, $-CH(CH_3)C(CH_3)_2-$, $-C(CH_3)_2CH_2-$, $C(CH_3)_2CH-(CH_3)-$, $C(CH_3)_2C(CH_3)_2-$, $-CH_2CH_2CH_2-$, $-CH_2CH_2CH_2CH_2-$, $-CH_2CH_2CH_2CH_2CH_2-$ and each of the last three groups in which one or more of the hydrogen atoms is replaced by a group or groups (which may be the same or different) selected from $CH_3$, $C_2H_5$, $C_3H_7$ (all isomers), $C_4H_9$ (all isomers) and $C_5H_{11}$ (all isomers), for example the compound of Example 9 in which one of the hydrogen atoms in the $-(CH_2)_3-$ group is replaced by methyl.

**Claims for the Contracting States: BE CH DE FR GB IT LI LU NL SE**

1. A compound of the formula:

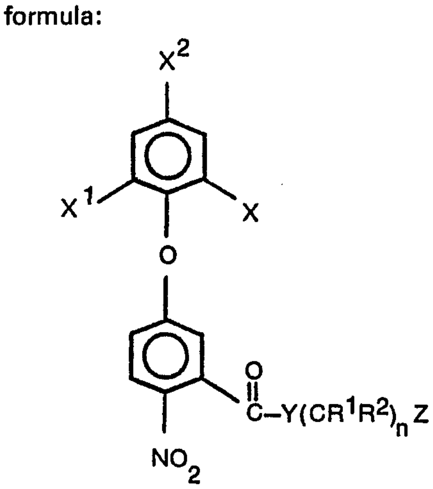

(I)

wherein

X is hydrogen, halo, trihalomethyl, alkyl, cyano or nitro;

$X^1$ is hydrogen, halo or trihalomethyl;

$X^2$ is trihalomethyl;

Y is O, NH, $NR^1$ or S;

$R^1$ and $R^2$ are the same or different and are selected from hydrogen, $(C_1-C_4)$alkyl and phenyl-$(C_1-C_4)$alkyl;

n is an integer of from 1 to 5, the $-CR^1R^2-$ groups being the same or different when n is greater than one; and

Z is carboxy, amino, mono$(C_1-C_4)$alkylamino, or di$(C_1-C_4)$alkylamino; and (when Z is carboxy) agronomically acceptable salts, esters and amides of a compound of Formula I.

2. A compound as claimed in Claim 1, and which is of the formula:

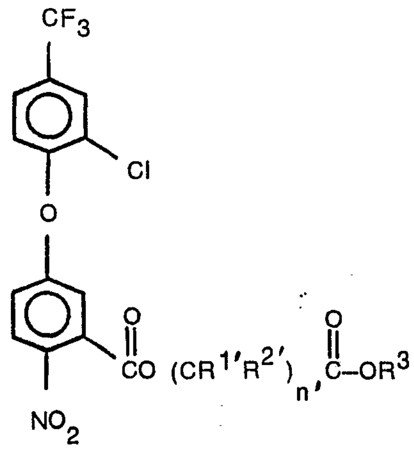

wherein

$R^{1'}$ and $R^{2'}$ are the same or different and are selected from hydrogen and $(C_1—C_4)$alkyl;
n' is 1 or 2; and
$R^3$ is $(C_1—C_4)$alkyl.

3. A compound as claimed in Claim 1, being a compound wherein (a) X is chlorine, $X^1$ is hydrogen, $X^2$ is trifluoromethyl, Y is oxygen, $R^1$ and $R^2$ are hydrogen, n is 1 and Z is $CO_2CH_3$, $CO_2$-$t$-$C_4H_9$, $CO_2H$, $CO_2CH_2CH_2OCH_3$, $CO_2CH_2CF_3$, $CO_2CH_2CH{=}CH_2$ or $CON(CH_3)_2$, (b) X is chlorine, $X^1$ is hydrogen, $X^2$ is trifluoromethyl, Y is oxygen, $R^1$ is methyl, $R^2$ is hydrogen, n is 1 and Z is $CO_2C_2H_5$ (c) X is chlorine, $X^1$ is hydrogen, $X^2$ is trifluoromethyl, Y is oxygen, $R^1$ and $R^2$ are hydrogen, n is 3 and Z is $CO_2C_2H_5$ or (d) X is chlorine, $X^1$ is hydrogen, $X^2$ is trifluoromethyl, Y is oxygen, $R^1$ and $R^2$ are methyl, n is 1 and Z is $CO_2C_2H_5$.

4. A compound as claimed in Claim 1, wherein X is chlorine, $X^1$ is hydrogen, $X^2$ is trifluoromethyl, Y is oxygen, $R^1$ is $CH_3$, $R^2$ is H, n is 1 and Z is $CO_2CH_3$.

5. A compound as claimed in Claim 1, wherein (a) X is chlorine, $X^1$ is hydrogen, $X^2$ is trifluoromethyl, Y is oxygen, $R^1$ and $R^2$ are hydrogen, n is 1 and Z is $CO_2Na$, $CO_2K$ or $CO_2Li$, (b) X is chlorine, $X^1$ is hydrogen, $X^2$ is trifluoromethyl, Y is oxygen, $R^1$ is benzyl, $R^2$ is hydrogen, n is 1 and Z is $CO_2CH_3$, (c) X is chlorine, $X^1$ is hydrogen, $X^2$ is trifluoromethyl, Y is O, S or NH, $R^1$ is hydrogen (when Y is S), $C_2H_5$ (when Y is O) or $CH_3$ or $i$-$C_3H_7$ (when Y is NH), $R^2$ is hydrogen, n is 1 and Z is $CO_2C_2H_5$, (d) X is chlorine, $X^1$ is hydrogen, $X^2$ is trifluoromethyl, Y is oxygen, $R^1$ is methyl, $R^2$ is hydrogen, n is 1 and Z is $CONH_2$, $CO_2H$, $CO_2Na$ or $CO_2K$, or (e) X is chlorine, $X^1$ is hydrogen, $X^2$ is trifluoromethyl, Y is oxygen, $—(CR^1R^2)_n—$ is $—(CH_2)_4—$ and Z is $CO_2C_2H_5$.

6. A compound as claimed in Claim 3, wherein $—(CR^{1'}R^{2'})_{n'}—$ is $—CH_2—$ or $—CH(CH_3)CH_2—$ and $R^3$ is $C_2H_5$.

7. A compound as claimed in Claim 1, wherein X is chlorine, $X^1$ is hydrogen, $X^2$ is trifluoromethyl, Y is oxygen and Z is $CO_2CH_3$ or $CO_2C_2H_5$, when Z is $CO_2CH_3$, $—(CR^1R^2)_n—$ being $—(CH_2)_2—$ or $—CH(CH_3)CH_2—$ and, when Z is $—CO_2C_2H_5$, $—CR^1R^2)_n—$ being $—C(CH_3)_2CH_2—$, $—CH(CH_3)CH(CH_3)—$, $—(CH_2)_2—$ or $—(CH_2)_3—$ in which one of the hydrogen atoms is replaced by $CH_3$.

8. A herbicidal composition containing, as active ingredient, at least one compound as claimed in any one of Claims 1 to 7 and an agronomically acceptable carrier or diluent for the active ingredient, the composition also optionally containing a surfactant.

9. A method of combating weeds which comprises applying to growing weeds or to the surface of a growth medium prior to emergence of weeds therefrom a compound as claimed in any one of Claims 1 to 7 in an amount sufficient to combat the growth of the weeds.

**Claims for the Contracting State: AT**

1. A herbicidal composition containing an active herbicidal component and an agronomically acceptable carrier or diluent therefor, wherein the active component comprises one or more diphenyl ethers of the formula:

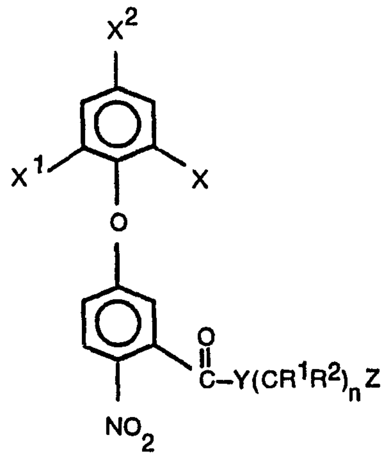

(I)

wherein

X is hydrogen, halo, trihalomethyl, alkyl, cyano or nitro;

$X^1$ is hydrogen, halo or trihalomethyl;

$X^2$ is trihalomethyl;

Y is O, NH, $NR^1$ or S;

$R^1$ and $R^2$ are the same or different and are selected from hydrogen, $(C_1—C_4)$alkyl and phenyl-$(C_1—C_4)$alkyl;

n is an integer of from 1 to 5, the $—CR^1R^2—$ groups being the same or different when n is greater than one; and

Z is carboxy, amino, mono$(C_1—C_4)$alkylamino, or di$(C_1—C_4)$alkylamino; and (when Z is carboxy) agronomically acceptable salts, esters and amides of a compound of Formula I.

2. A composition as claimed in Claim 1, wherein the diphenyl ether is of the formula

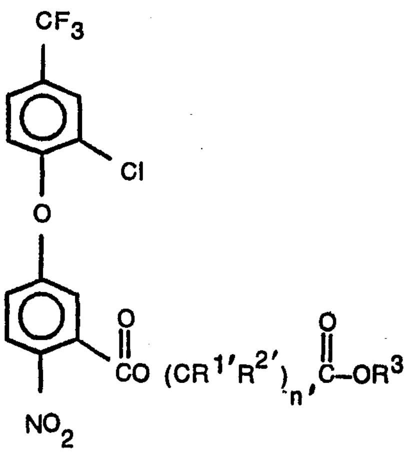

wherein

$R^{1'}$ and $R^{2'}$ are the same or different and are selected from hydrogen and $(C_1—C_4)$alkyl;

n' is 1 or 2; and

$R^3$ is $(C_1—C_4)$alkyl.

3. A composition as claimed in Claim 1, being a compound wherein (a) X is chlorine, $X^1$ is hydrogen, $X^2$ is trifluoromethyl, Y is oxygen, $R^1$ and $R^2$ are hydrogen, n is 1 and Z is $CO_2CH_3$, $CO_2$-$t$-$C_4H_9$, $CO_2H$, $CO_2CH_2CH_2OCH_3$, $CO_2CH_2CF_3$, $CO_2CH_2CH{=}CH_2$ or $CON(CH_3)_2$, (b) X is chlorine, $X^1$ is hydrogen, $X^2$ is trifluoromethyl, Y is oxygen, $R^1$ is methyl, $R^2$ is hydrogen, n is 1 and Z is $CO_2C_2H_5$ (c) X is chlorine, $X^1$ is hydrogen, $X^2$ is trifluoromethyl, Y is oxygen, $R^1$ and $R^2$ are hydrogen, n is 3 and Z is $CO_2C_2H_5$ or (d) X is chlorine, $X^1$ is hydrogen, $X^2$ is trifluoromethyl, Y is oxygen, $R^1$ and $R^2$ are methyl, n is 1 and Z is $CO_2C_2H_5$.

4. A composition as claimed in Claim 1, wherein X is chlorine, $X^1$ is hydrogen, $X^2$ is trifluoromethyl, Y is oxygen, $R^1$ is $CH_3$, $R^2$ is H, n is 1 and Z is $CO_2CH_3$.

5. A composition as claimed in Claim 1, wherein (a) X is chlorine, $X^1$ is hydrogen, $X^2$ is trifluoromethyl, Y is oxygen, $R^1$ and $R^2$ are hydrogen, n is 1 and Z is $CO_2Na$, $CO_2K$ or $CO_2Li$, (b) X is chlorine, $X^1$ is

hydrogen, $X^2$ is trifluoromethyl, Y is oxygen, $R^1$ is benzyl, $R^2$ is hydrogen, n is 1 and Z is $CO_2CH_3$, (c) X is chlorine, $X^1$ is hydrogen, $X^2$ is trifluoromethyl, Y is O, S or NH, $R^1$ is hydrogen (when Y is S), $C_2H_5$ (when Y is O) or $CH_3$ or $i$-$C_3H_7$ (when Y is NH), $R^2$ is hydrogen, n is 1 and Z is $CO_2C_2H_5$, (d) X is chlorine, $X^1$ is hydrogen, $X^2$ is trifluoromethyl, Y is oxygen, $R^1$ is methyl, $R^2$ is hydrogen, n is 1 and Z is $CONH_2$, $CO_2H$, $CO_2Na$ or $CO_2K$, or (e) X is chlorine, $X^1$ is hydrogen, $X^2$ is trifluoromethyl, Y is oxygen, $-(CR^1R^2)_n-$ is $-(CH_2)_4-$ and Z is $CO_2C_2H_5$.

6. A composition as claimed in Claim 3, wherein $-(CR^{1'}R^{2'})_{n'}-$ is $-CH_2-$ or $-CH(CH_3)CH_2-$ and $R^3$ is $C_2H_5$.

7. A composition as claimed in Claim 1, wherein X is chlorine, $X^1$ is hydrogen, $X^2$ is trifluoromethyl, Y is oxygen and Z is $CO_2CH_3$ or $CO_2C_2H_5$, when Z is $CO_2CH_3$, $-(CR^1R^2)_n-$ being $-(CH_2)_2-$ or $-CH(CH_3)CH_2-$ and, when Z is $-CO_2C_2H_5$, $-(CR^1R^2)_n-$ being $-C(CH_3)_2CH_2-$, $-CH(CH_3)CH(CH_3)-$, $-(CH_2)_2-$ or $-(CH_2)_3-$ in which one of the hydrogen atoms is replaced by $CH_3$.

8. A method of combating weeds which comprises applying to growing weeds or to the surface of a growth medium prior to emergence of weeds therefrom a composition as claimed in any one of Claims 1 to 7 in an amount sufficient to combat the growth of the weeds.

**Revendications pour les Etats contractants: BE CH DE FR GB IT LI LU NL SE**

1. Composé représentable par la formule:

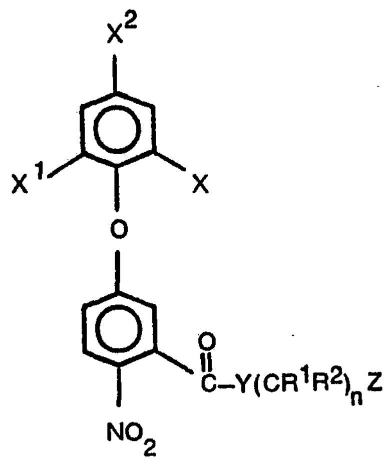

(I)

dans laquelle:

X est hydrogène, halogéno, trihalogénométhyle, alcoyle, cyano ou nitro;

$X^1$ est hydrogène, halogéno ou trihalogénométhyle;

$X^2$ est trihalogénométhyle;

Y est O, NH, $NR^1$ ou S;

$R^1$ et $R^2$ sont identiques ou différents et sont choisis parmi hydrogène, alcoyle en $C_1$ à $C_4$ et phényl-alcoyle en $C_1$ à $C_4$;

n est un nombre entier de 1 à 5, les radicaux $-CR^1R^2-$ étant identiques ou différents quand n est plus grand que un; et

Z est carboxy, amino, mono-alcoyl(en $C_1$ à $C_4$)amino, ou di-alcoyl(en $C_1$ à $C_4$)amino; et (quand Z est carboxy) des sels, esters et amides agronomiquement acceptables d'un composé représentable par la Formule (I).

2. Composé selon la revendication 1, et qui possède la formule suivante:

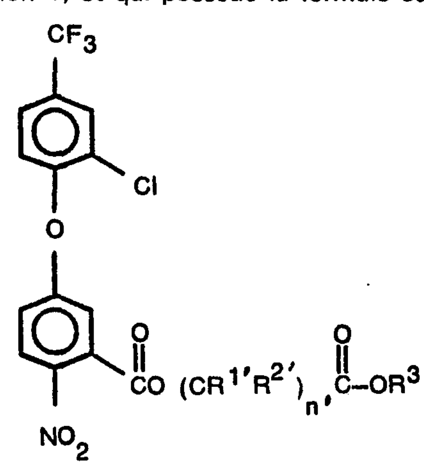

dans laquelle

$R^{1'}$ et $R^{2'}$ sont identiques ou différents et sont choisis parmi hydrogène et alcoyle en $C_1$ à $C_4$;

n' est 1 ou 2; et

$R^3$ est alcoyle en $C_1$ à $C_4$.

3. Composé selon la revendication 1, composé dans lequel (a) X est chlore, $X^1$ est hydrogène, $X^2$ est trifluorométhyle, Y est oxygène, $R^1$ et $R^2$ sont hydrogène, n est 1, et Z est $CO_2CH_3$, $CO_2$-*tert*-$C_4H_9$, $CO_2H$, $CO_2CH_2CH_2OCH_3$, $CO_2CH_2CF_3$, $CO_2CH_2CH{=}CH_2$ ou $CON(CH_3)_2$, (b) X est chlore, $X^1$ est hydrogène, $X^2$ est trifluorométhyle, Y est oxygène, $R^1$ est méthyle, $R^2$ est hydrogène, n est 1, et Z est $CO_2C_2H_5$, (c) X est chlore, $X^1$ est hydrogène, $X^2$ est trifluorométhyle, Y est oxygène, $R^1$ et $R^2$ sont hydrogène, n est 3 et Z est $CO_2C_2H_5$, ou bien (d) X est chlore, $X^1$ est hydrogène, $X^2$ est trifluorométhyle, Y est oxygène, $R^1$ et $R^2$ sont méthyle, n est 1, et Z est $CO_2C_2H_5$.

4. Composé selon la revendication 1, et dans lequel X est chlore, $X^1$ est hydrogène, $X^2$ est trifluorométhyle, Y est oxygène, $R^1$ est $CH_3$, $R^2$ est H, n est 1, et Z est $CO_2CH_3$.

5. Composé selon la revendication 1, et dans lequel (a) X est chlore, $X^1$ est hydrogène, $X^2$ est trifluorométhyle, Y est oxygène, $R^1$ et $R^2$ sont hydrogène, n est 1 et Z est $CO_2Na$, $CO_2K$ ou $CO_2Li$, (b) X est chlore, $X^1$ est hydrogène, $X^2$ est trifluorométhyle, Y est oxygène, $R^1$ est benzyle, $R^2$ est hydrogène, n est 1 et Z est $CO_2CH_3$, (c) X est chlore, $X^1$ est hydrogène, $X^2$ est trifluorométhyle, Y est O, S ou NH, $R^1$ est hydrogène (quand Y est S), $C_2H_5$ (quand Y est O) ou bien $CH_3$ ou *i*-$C_3H_7$ (quand Y est NH), $R^2$ est hydrogène, n est 1, et Z est $CO_2C_2H_5$, (d) X est chlore, $X^1$ est hydrogène, $X^2$ est trifluorométhyle, Y est oxygène, $R^1$ est méthyle, $R^2$ est hydrogène, n est 1 et Z est $CONH_2$, $CO_2H$, $CO_2Na$ ou $CO_2K$, ou bien (e) X est chlore, $X^1$ est hydrogène, $X^2$ est trifluorométhyle, Y est oxygène, $-(CR^1R^2)_n-$ est $-(CH_2)_4-$, et Z est $CO_2C_2H_5$.

6. Composé selon la revendication 3, et dans lequel $-(CR^{1'}R^{2'})_{n'}-$ est $-CH_2-$ ou $-CH(CH_3)CH_2-$, et $R^3$ est $C_2H_5$.

7. Composé selon la revendication 1, et dans lequel X est chlore, $X^1$ est hydrogène, $X^2$ est trifluorométhyle, Y est oxygène, et Z est $CO_2CH_3$ ou $CO_2C_2H_5$, quand Z est $CO_2CH_3$, $-(CR^1R^2)_n-$ étant $-(CH_2)_2-$ ou $-CH(CH_3)CH_2-$ et, quand Z est $CO_2C_2H_5$, $-(CR^1R^2)_n-$ étant $-C(CH_3)_2CH_2-$, $-CH(CH_3)CH(CH_3)-$, $-(CH_2)_2-$ ou $-(CH_2)_3$ dans lequel un des atomes d'hydrogène est remplacé par $CH_3$.

8. Composition herbicide contenant, comme ingrédient actif, au moins un composé selon l'une quelconque des revendications 1 à 7 et un véhicule ou diluant agronomiquement acceptable pour l'ingrédient actif, la composition contenant aussi, facultativement, un agent tensio-actif.

9. Procédé pour combattre des mauvaises herbes et qui comprend l'application, aux mauvaises herbes en cours de croissance ou à la surface d'un milieu de culture avant que les mauvaises herbes en sortent, d'un composé selon l'une quelconque des revendications 1 à 7 en une proportion suffisante pour combattre la croissance des mauvaises herbes.

**Revendications pour l'Etat contractant: AT**

1. Composition herbicide contenant un composé herbicide actif et un diluant ou un véhicule agronomiquement acceptable pour le composé actif, dans laquelle le composé actif comprend un ou plusieurs éthers diphényle de formule:

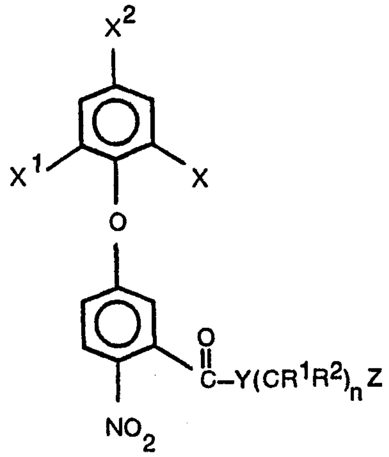

(I)

dans laquelle:

X est hydrogène, halogéno, trihalogénométhyle, alcoyle, cyano ou nitro;

$X^1$ est hydrogène, halogéno ou trihalogénométhyle;

$X^2$ est trihalogénométhyle;

Y est O, NH, $NR^1$ ou S;

$R^1$ et $R^2$ sont identiques ou différents et sont choisis parmi hydrogène, alcoyle en $C_1$ à $C_4$ et phényl-alcoyle en $C_1$ à $C_4$;

n est un nombre entier de 1 à 5, les radicaux $—CR^1R^2—$ étant identiques ou différents quand n est plus grand que un; et

Z est carboxy, amino, mono-alcoyl(en $C_1$ à $C_4$)amino, ou di-alcoyl(en $C_1$ à $C_4$)amino; et (quand Z est carboxy) des sels, esters et amides agronomiquement acceptables d'un composé représentable par la Formule (I).

2. Composition selon la revendication 1, dans laquelle l'éther diphényle a la formule suivante:

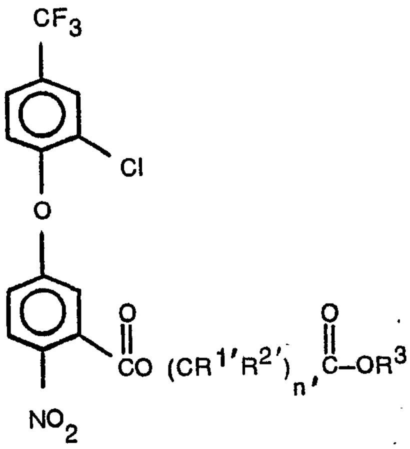

dans laquelle

$R^{1'}$ et $R^{2'}$ sont identiques ou différents et sont choisis parmi hydrogène et alcoyle en $C_1$ à $C_4$;

n' est 1 ou 2; et

$R^3$ est alcoyle en $C_1$ à $C_4$.

3. Composition selon la revendication 1, étant un composé dans lequel (a) X est chlore, $X^1$ est hydrogène, $X^2$ est trifluorométhyle, Y est oxygène, $R^1$ et $R^2$ sont hydrogène, n est 1, et Z est $CO_2CH_3$, $CO_2$-*tert*-$C_4H_9$, $CO_2H$, $CO_2CH_2CH_2OCH_3$, $CO_2CH_2CF_3$, $CO_2CH_2CH{=}CH_2$ ou $CON(CH_3)_2$, (b) X est chlore, $X^1$ est hydrogène, $X^2$ est trifluorométhyle, Y est oxygène, $R^1$ est méthyle, $R^2$ est hydrogène, n est 1, et Z est $CO_2C_2H_5$, (c) X est chlore, $X^1$ est hydrogène, $X^2$ est trifluorométhyle, Y est oxygène, $R^1$ et $R^2$ sont hydrogène, n est 3 et Z est $CO_2C_2H_5$, ou bien (d) X est chlore, $X^1$ est hydrogène, $X^2$ est trifluorométhyle, Y est oxygène, $R^1$ et $R^2$ sont méthyle, n est 1, et Z est $CO_2C_2H_5$.

4. Composition selon la revendication 1, et dans lequel X est chlore, $X^1$ est hydrogène, $X^2$ est trifluorométhyle, Y est oxygène, $R^1$ est $CH_3$, $R^2$ est H, n est 1, et Z est $CO_2CH_3$.

5. Composition selon la revendication 1, et dans lequel (a) X est chlore, $X^1$ est hydrogène, $X^2$ est trifluorométhyle, Y est oxygène, $R^1$ et $R^2$ sont hydrogène, n est 1 et Z est $CO_2Na$, $CO_2K$ ou $CO_2Li$, (b) X est chlore, $X^1$ est hydrogène, $X^2$ est trifluorométhyle, Y est oxygène, $R^1$ est benzyle, $R^2$ est hydrogène, n est 1 et Z est $CO_2CH_3$, (c) X est chlore, $X^1$ est hydrogène, $X^2$ est trifluorométhyle, Y est O, S ou NH, $R^1$ est hydrogène (quand Y est S), $C_2H_5$ (quand Y est O) ou bien $CH_3$ ou *i*-$C_3H_7$ (quand Y est NH), $R^2$ est hydrogène, n est 1, et Z est $CO_2C_2H_5$, (d) X est chlore, $X^1$ est hydrogène, $X^2$ est trifluorométhyle, Y est oxygène, $R^1$ est méthyle, $R^2$ est hydrogène, n est 1 et Z est $CONH_2$, $CO_2H$, $CO_2Na$ ou $CO_2K$, ou bien (e) X est chlore, $X^1$ est hydrogène, $X^2$ est trifluorométhyle, Y est oxygène, $—(CR^1R^2)_n—$ est $—(CH_2)_4—$, et Z est $CO_2C_2H_5$.

6. Composition selon la revendication 3, dans laquelle $—(CR^{1'}R^{2'})_{n'}—$ est $—CH_2—$ ou $—CH(CH_3)CH_2—$, et $R^3$ est $C_2H_5$.

7. Composition selon la revendication 1, dans laquelle X est chlore, $X^1$ est hydrogène, $X^2$ est trifluorométhyle, Y est oxygène, et Z est $CO_2CH_3$ ou $CO_2C_2H_5$, quand Z est $CO_2CH_3$, $—(CR^1R^2)_n—$ étant $—(CH_2)_2—$ ou $—CH(CH_3)CH_2—$ et, quand Z est $CO_2C_2H_5$, $—(CR^1R^2)_n—$ étant $—C(CH_3)_2CH_2—$, $—CH(CH_3)CH(CH_3)—$, $—(CH_2)_2—$ ou $—(CH_2)_3$ dans lequel un des atomes d'hydrogène est remplacé par $CH_3$.

8. Procédé pour combattre les mauvaises herbes et qui comprend l'application, aux mauvaises herbes en cours de croissance ou à la surface d'un milieu de culture avant que les mauvaises herbes en sortent, d'une composition selon l'une quelconque des revendications 1 à 7 en une proportion suffisante pour combattre la croissance des mauvaises herbes.

**Patentansprüche für die Vertragsstaaten: BE CH DE FR GB IT LI LU NL SE**

1. Verbindung der Formel

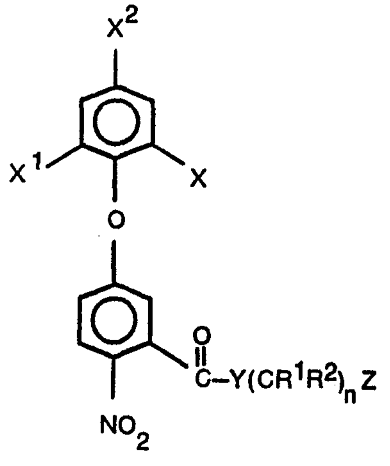

(I)

worin

X für Wasserstoff, Halogen, Trihalogenmethyl, Alkyl, Cyano oder Nitro steht,

$X^1$ Wasserstoff, Halogen oder Trihalogenmethyl ist,

$X^2$ Trihalogenmethyl bedeutet,

Y O, NH, $NR^1$ oder S ist,

$R^1$ und $R^2$ gleich oder verschieden sind und aus Wasserstoff, $(C_1-C_4)$Alkyl und Phenyl$(C_1-C_4)$-alkyl ausgewählt werden,

n eine ganze Zahl von 1 bis 5 ist, wobei die $-CR^1R^2-$ Gruppen gleich oder verschieden sind, wenn n größer als 1 ist und

Z für Carboxy, Amino, Mono$(C_1-C_4)$alkylamino oder Di$(C_1-C_4)$alkylamino steht, und, falls Z Carboxy bedeutet, für landwirtschaftliche Zwecke verträgliche Salze, Ester und Amide einer Verbindung der Formel I.

2. Verbindung nach Anspruch 1, dadurch gekennzeichnet, daß sie der Formel

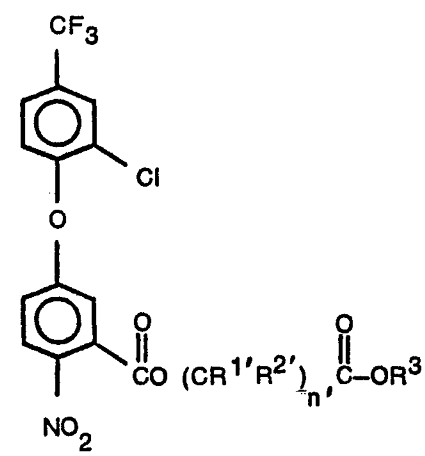

entspricht, worin

$R^{1'}$ und $R^{2'}$ gleich oder verschieden sind und aus Wasserstoff und $(C_1-C_4)$Alkyl ausgewählt werden,

n' 1 oder 2 ist und

$R^3$ $(C_1-C_4)$Alkyl bedeutet.

3. Verbindung nach Anspruch 1, dadurch gekennzeichnet, daß (a) X für Chlor steht, $X^1$ Wasserstoff bedeutet, $X^2$ Trifluormethyl ist, Y Sauerstoff ist, $R^1$ und $R^2$ Wasserstoff sind, n 1 ist und Z $CO_2CH_3$, $CO_2$-t-$C_4H_9$, $CO_2H$, $CO_2CH_2CH_2OCH_3$, $CO_2CH_2CF_3$, $CO_2CH_2{=}CH_2$ oder $CON(CH_3)_2$ ist, (b) X für Chlor steht, $X^1$ Wasserstoff ist, $X^2$ Trifluormethyl ist, Y Sauerstoff ist, $R^1$ Methyl ist, $R^2$ Wasserstoff ist, n 1 ist und Z $CO_2C_2H_5$ bedeutet, (c) X für Chlor steht, $X^1$ Wasserstoff ist, $X^2$ Trifluormethyl bedeutet, Y Sauerstoff ist, $R^1$ und $R^2$ Wasserstoff sind, n 3 ist und Z $CO_2C_2H_5$ bedeutet und (d) X für Chlor steht, $X^1$ Wasserstoff bedeutet, $X^2$ Trifluormethyl ist, Y Sauerstoff ist, $R^1$ und $R^2$ Methyl sind und n 1 bedeutet und Z für $CO_2C_2H_5$ steht.

4. Verbindung nach Anspruch 1, dadurch gekennzeichnet, daß X für Chlor steht, $X^1$ Wasserstoff bedeutet, $X^2$ Trifluormethyl ist, Y Sauerstoff bedeutet, $R^1$ $CH_3$ ist, $R^2$ H ist, n 1 ist und Z $CO_2CH_3$ ist.

5. Verbindung nach Anspruch 1, dadurch gekennzeichnet, daß (a) X für Chlor steht, $X^1$ Wasserstoff ist, $X^2$ Trifluormethyl ist, Y Sauerstoff ist, $R^1$ und $R^2$ Wasserstoff sind, n 1 ist und Z $CO_2Na$ oder $CO_2K$ oder $CO_2Li$ ist, (b) X Chlor ist, $X^1$ Wasserstoff ist, $X^2$ Trifluormethyl ist, Y Sauerstoff ist, $R^1$ Benzyl ist, $R^2$ Wasserstoff ist, n 1 ist und Z $CO_2CH_3$, (c) X Chlor bedeutet, $X^1$ Wasserstoff ist, $X^2$ Trifluormethyl darstellt, Y O, S oder NH bedeutet, $R^1$ Wasserstoff (falls Y S bedeutet), $C_2H_5$ (falls Y O ist) oder $CH_3$ oder i-$C_3H_7$ (falls Y NH bedeutet) ist, $R^2$ Wasserstoff ist, n 1 ist und Z $CO_2C_2H_5$ ist, (d) X Chlor ist, $X^1$ Wasserstoff ist, $X^2$ Trifluormethyl ist, Y Sauerstoff ist, $R^1$ Methyl ist, $R^2$ Wasserstoff ist, n 1 ist und Z $CONH_2$, $CO_2H$, $CO_2Na$ oder $CO_2K$ ist, oder (e) X Chlor bedeutet, $X^1$ Wasserstoff ist, $X^2$ Trifluormethyl ist, Y Sauerstoff oder $-(CR^1R^2)_n-$ — $(CH_2)_4-$ ist und Z $CO_2C_2H_5$ bedeutet.

6. Verbindung nach Anspruch 3, dadurch gekennzeichnet, daß $-(CR^{1'}R^{2'})_{n'}-$ für $-CH_2-$ oder $-CH(CH_3)CH_2-$ steht und $R^3$ $C_2H_5$ ist.

7. Verbindung nach Anspruch 1, dadurch gekennzeichnet, daß X für Chlor steht, $X^1$ Wasserstoff bedeutet, $X^2$ Trifluormethyl ist, Y Sauerstoff darstellt und Z $CO_2CH_3$ oder $CO_2C_2H_5$ ist, falls Z $CO_2CH_3$ ist, $-(CR^1R^2)_{n'}-$ für $-(CH_2)_2-$ oder $-CH(CH_3)CH_2-$ steht und, falls Z $-CH_2C_2H_5$ ist, $-(CR^1R^2)_n-$ für $-C(CH_3)_2CH_2-$, $-CH(CH_3)CH(CH_3)-$, $-(CH_2)_2-$ oder $-(CH_2)_3-$ steht, wobei eines der Wasserstoffatome durch $CH_3$ ersetzt ist.

8. Herbizides Mittel, dadurch gekennzeichnet, daß es als Wirkstoff wenigstens eine Verbindung gemäß einem der Ansprüche 1 bis 7 sowie einen für landwirtschaftliche Zwecke verträglichen Träger oder Verdünnungsmittel für den Wirkstoff enthält, wobei das Mittel außerdem gegebenenfalls ein grenzflächenaktives Mittel enthält.

9. Verfahren zur Bekämpfung von Unkräutern, dadurch gekennzeichnet, daß auf die wachsenden Unkräuter oder auf die Oberfläche eines Wachstumsmediums vor dem Auflaufen der Unkräuter eine Verbindung gemäß einem der Ansprüche 1 bis 7 in einer Menge aufgebracht wird, die dazu ausreicht, das Wachstum der Unkräuter zu bekämpfen.

**Patentansprüche für den Vertragsstaat: AT**

1. Herbizides Mittel, das eine aktive herbizide Komponente und ein für landwirtschaftliche Zwecke verträgliches Verdünnungsmittel oder einen für landwirtschaftliche Zwecke verträglichen Träger enthält, dadurch gekennzeichnet, daß die aktive Komponente aus einem oder mehreren Diphenylethern der Formel

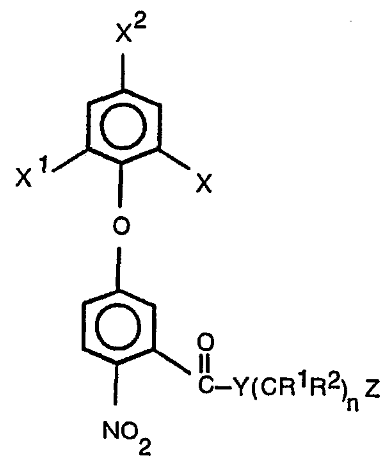

(I)

worin

X für Wasserstoff, Halogen, Trihalogenmethyl, Alkyl, Cyano oder Nitro steht,

$X^1$ Wasserstoff, Halogen oder Trihalogenmethyl ist,

$X^2$ Trihalogenmethyl ist,

Y O, NH, $NR^1$ oder S ist,

$R^1$ und $R^2$ gleich oder verschieden sind und aus Wasserstoff, $(C_1-C_4)$Alkyl und Phenyl$(C_1-C_4)$-alkyl ausgewählt sind.

n eine ganze Zahl von 1 bis 5 ist, wobei die $-CR^1R^2-$ Gruppen gleich oder verschieden sind, wenn n größer als 1 ist, und

Z Carboxy, Amino, Mono$(C_1-C_4)$alkylamino oder Di$(C_1-C_4)$alkylamino ist, und (falls Z für Carboxy steht) aus für landwirtschaftliche Zwecke verträglichen Salzen, Estern und Amiden einer Verbindung der Formel I besteht.

2. Mittel nach Anspruch 1, dadurch gekennzeichnet, daß der Diphenylether der Formel

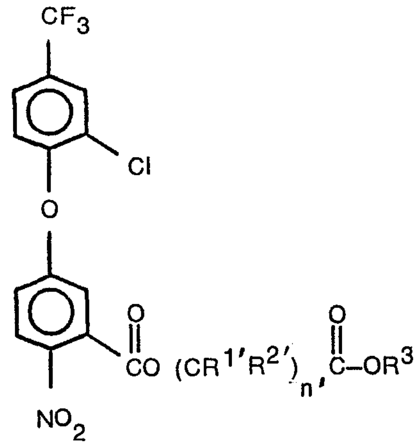

entspricht, worin

$R^{1'}$ und $R^{2'}$ gleich oder verschieden sind und aus Wasserstoff oder $(C_1—C_4)$Alkyl ausgewählt werden,

n' 1 oder 2 ist und

$R^3$ $(C_1—C_4)$Alkyl bedeutet.

3. Mittel nach Anspruch 1, dadurch gekennzeichnet, daß in dem Wirkstoff (a) X für Chlor steht, $X^1$ Wasserstoff bedeutet, $X^2$ Trifluoromethyl ist, Y Sauerstoff ist, $R^1$ und $R^2$ Wasserstoff sind, n 1 bedeutet und Z $CO_2CH_3$, $CO_2$-t-$C_4H_9$, $CO_2H$, $CO_2CH_2CH_2OCH_3$, $CO_2CH_2CF_3$, $CO_2CH_2CH{=}CH_2$ oder $CON(CH_3)_2$ ist, (b) X für Chlor steht, $X^1$ Wasserstoff ist, $X^2$ Trifluoromethyl ist, Y Sauerstoff ist, $R^1$ Methyl ist, $R^2$ Wasserstoff ist, n 1 ist und Z $CO_2C_2H_5$ bedeutet, (c) X für Chlor steht, $X^1$ Wasserstoff ist, $X^2$ Trifluormethyl bedeutet, Y Sauerstoff ist, $R^1$ und $R^2$ Wasserstoff sind, n 3 ist und Z $CO_2C_2H_5$ bedeutet und (d) X für Chlor steht, $X^1$ Wasserstoff bedeutet, $X^2$ Trifluoromethyl ist, Y Sauerstoff ist, $R^1$ und $R^2$ Methyl sind und n 1 bedeutet und Z für $CO_2C_2H_5$ steht.

4. Mittel nach Anspruch 1, dadurch gekennzeichnet, daß X für Chlor steht, $X^1$ Wasserstoff bedeutet, $X^2$ Trifluormethyl ist, Y Sauerstoff bedeutet, $R^1$ $CH_3$ ist, $R^2$ H ist, n 1 ist und Z $CO_2CH_3$ ist.

5. Mittel nach Anspruch 1, dadurch gekennzeichnet, daß (a) X für Chlor steht, $X^1$ Wasserstoff ist, $X^2$ Trifluormethyl ist, Y Sauerstoff ist, $R^1$ und $R^2$ Wasserstoff sind, n 1 ist und Z $CO_2Na$ oder $CO_2K$ oder $CO_2Li$ ist, (b) X Chlor ist, $X^1$ Wasserstoff ist, $X^2$ Trifluormethyl ist, Y Sauerstoff ist, $R^1$ Benzyl ist, $R^2$ Wasserstoff ist, n 1 ist und Z $CO_2CH_3$, (c) X Chlor bedeutet, $X^1$ Wasserstoff ist, $X^2$ Trifluormethyl darstellt, Y O, S oder NH bedeutet, $R^1$ Wasserstoff (falls Y S bedeutet), $C_2H_5$ (falls Y O ist) oder $CH_3$ oder i-$C_3H_7$ (falls Y NH bedeutet) ist, $R^2$ Wasserstoff ist, n 1 ist und Z $CO_2C_2H_5$ ist, (d) X Chlor ist, $X^1$ Wasserstoff ist, $X^2$ Trifluormethyl ist, Y Sauerstoff ist, $R^1$ Methyl ist, $R^2$ Wasserstoff ist, n 1 ist und Z $CONH_2$, $CO_2H$, $CO_2Na$ oder $CO_2K$ ist, oder (e) X Chlor bedeutet, $X^1$ Wasserstoff ist, $X^2$ Trifluormethyl ist, Y Sauerstoff oder $—(CR^1R^2)_n—$ — $(CH_2)_4—$ ist und Z $CO_2C_2H_5$ bedeutet.

6. Mittel nach Anspruch 3, dadurch gekennzeichnet, daß $—(CR^{1'}R^{2'})_{n'}—$ für $—CH_2—$ oder $—CH(CH_3)CH_2—$ steht und $R^3$ $C_2H_5$ ist.

7. Mittel nach Anspruch 1, dadurch gekennzeichnet, daß X für Chlor steht, $X^1$ Wasserstoff bedeutet, $X^2$ Trifluormethyl ist, Y Sauerstoff darstellt und Z $CO_2CH_3$ oder $CO_2C_2H_5$ ist, falls Z $CO_2CH_3$ ist, $—(CR^1R^2)_{n'}—$ für $—(CH_2)_2—$ oder $—CH(CH_3)CH_2—$ steht und, falls Z $—CH_2C_2H_5$ ist, $—(CR^1R^2)_n—$ für $—C(CH_3)_2CH_2—$, $—CH(CH_3)CH(CH_3)—$, $—(CH_2)_2—$ oder $—(CH_2)_3—$ steht, wobei eines der Wasserstoffatome durch $CH_3$ ersetzt ist.

8. Verfahren zur Bekämpfung von Unkräutern, dadurch gekennzeichnet, daß auf die wachsenden Unkräuter oder auf die Oberfläche eines Wachstumsmediums vor dem Auflaufen der Unkräuter ein Mittel gemäß einem der Ansprüche 1 bis 7 in einer Menge aufgebracht wird, die zur Bekämpfung des Wachstums der Unkräuter ausreicht.